# EUROPEAN PATENT APPLICATION

(11) **EP 2 359 850 A1**
(43) Date of publication of application: **24.08.2011**
(21) Application number: 09827196.8
(22) Date of filing: 19.11.2009
(51) Int. Cl.: A61K 39/095, A61K 9/127, A61P 37/04

(54) **SINGLE-TIME VACCINES**

(30) Priority: 19.11.2008 CU 20080215
(71) Applicant: Instituto Finlay, Centro de Investigacion-Produccion de vacunas y sueros, La Coronela La Lisa Ciudad Habana (CU)
(72) Inventor: PEREZ MARTIN, Oliver Germán, 11300 Ciudad de la Habana (CU); GONZALEZ AZNAR, Elizabeth, 11300 Ciudad de la Habana (CU); ROMEU ALVAREZ, Belkis, 13100 Ciudad de la Habana (CU); DEL CAMPO ALONSO, Judith Mónica, 10400 Ciudad de la Habana (CU); ACEVEDO GROGUES, Reinaldo, 11100 Ciudad de la Habana (CU); LASTRE GONZALEZ, Miriam de San Juan Bosco, 11300 Ciudad de la Habana (CU); ZAYAS VIGNIER, Caridad, 11400 Ciudad de la Habana (CU); CUELLO PEREZ, Maribel, 12100 Ciudad de la Habana (CU); CABRERA BLANCO, Osmir, 11600 Ciudad de la Habana (CU); NUÑEZ GUTIERREZ, Niurys, 11300 Ciudad de la Habana (CU); BALBOA GONZALEZ, Julio Antonio, 11300 Ciudad de la Habana (CU)
(74) Representative: Jansen, Cornelis Marinus
(86) International application number: PCT/CU2009/000008
(87) International publication number: WO 2010/057447

(57) **Abstract**

The invention is related to the field of vaccines. The technical objective is to obtain novel unitemporal therapeutic and prophylactic vaccines (single time vaccination strategy) using simultaneously one or more mucosal doses and one parenteral dose where the use of potent mucosal adjuvants are essential, preferable the Cochleate (AFCo1, Adjuvant Finlay Cochleate 1). The incorporation or co-administration by parenteral and mucosal routes of non-related PL antigens (heterologous), and/or the additional use of delivery systems like Aluminum, Chitosan or oil/water emulsions by parenteral application is also included in this invention. The induction of cellular immune responses than including systemic specific IgG and secretory specific IgA is obtained with at least two simultaneously doses. So, this unitemporal strategy is applicable to obtaining new unique or multiples vaccines (several mucosal vaccine antigens with the corresponding parenteral combine vaccine) in the pharmaceutical industry which permit to increase vaccine coverage and reduce the time-cost of vaccine campaigns.

## Description

This invention relates to the field of new vaccines, particularly in relation to the development of a novel immunization strategy (Single Time Vaccination Strategy) allowing the use of unitemporals vaccines for the treatment and prevention of infections diseases of different nature, including also tumor diseases.

More particularly, it relates to a vaccine strategy which solves the problems of inefficient multidose vaccination where the number of doses produces the absence and the discontinuous participation of the subject to subsequent doses, particularly important feature in developing countries where populations do not have an easy access to medical services and where localize and vaccinate a large population is very difficult. Likewise the new strategy makes the current parenteral vaccines more efficient, because are capable to induce mucosal responses too when are used combinations of potent mucosal and parenteral adjuvants with vaccine antigens, incorporated, conjugated or co-administered. The technical objective pursued is to increase vaccine coverage and to induce mucosal and parenteral combined responses against heterologous antigens (fungal, viral, protozoan, helminthic or carcinogenic) or against their own antigens, especially to induce secretory IgA response (SIgA) involved in the protection against germs and tumors that affect the mucous, ensuring further systemic response of specific IgG antibodies, also involved in mucosal protection. This extends to a better acceptance of the immunizations, by reducing the number of doses and the combined use of a new route of immunization, less reactogenic and much more accepted by the population as the mucosal route. These results lead to the development of unitemporals vaccine formulations for prophylactic and therapeutic use.

In this sense, arises in the new strategy, the use of proteoliposomes (AFPL1) and its Cochlear derivatives (AFCo1), obtained from any microorganism, as parenteral and mucosal adjuvants respectively, in formulations with heterologous antigens (fungal, viral, bacterial, protozoa, helminth or carcinogenic), inserted, conjugated or co-administrated in their structure or as vaccine candidates against self antigens present in the AFPL and its derivatives. As an adjuvant they have exceptional characteristics, combining in the same structure, the potentiator activity, polarizing agents and delivery system capacities, presenting multimeric protein copies; containing multiprotein composition and multi and synergistic PAMP components (LPS, porines and DNA traces). The immune response induced by these formulations against its own antigens is preferential T helper type 1 (Th1) with TCD4+, TCD8+, cross-presentation and cytotoxic T-lymphocyte (CTL) in vivo responses and the same pattern of response is extended to the antigens included, conjugated or co-administered to their structures administrated by parenteral or mucosal routes (Pérez O et al. Infect Immun. 2001, 69(7):4502-4508; Pérez O, et al. Scand J Immunology 2007,66:271-77). Also these structures induce an efficient mucosal immune response with specific secretory IgA response on mucosal secretions to be administered by mucosal route, although AFCo1 induce a significant higher immune response than AFPL1 (Pérez O et al. Immunology and cell Biology. 2004, 82: 603-610)

### State of the art of the invention

Vaccination is considered by the World Health Organization (WHO) to be the most cost-effective strategy for controlling infectious diseases and together with improvements in hygiene and sanitation, have had a tremendous impact on global public health. Immunizations, through immunization programs around the world, have been prevented more than 2.5 millions of death from infectious diseases in all age group every year. In spite of this in certain parts of the world, especially in developing countries, many people (mostly children), still die due to infectious diseases preventable by current vaccines. This is because although the vaccines coverage have been increased every year they are still inefficient. Several factors have been responsible of difficulty to attain immunization coverage. Some of them are general or demographic like poverty, health policies of the governments of each country, wars, among others; and others, known as the problems of current vaccines such as: the number of doses, excessive use of parenteral route, the reactogenicity, few adjuvants approved for human use, few combined vaccines, instability to heat during transportation and high economic cost.

The number of dose in the immunization schedule or the number of contacts needed to achieve full immunization schedule is one of the main problems of current vaccines. Only a few live vaccines like Polio have achieved this goal. In contrast with live vaccines, it has been difficult to obtain good immune response with a single dose by nonliving antigen vaccines, where booster doses is require in order to consolidate immunity against diseases. Localize and vaccinate a large population is very difficult in places where medical personal are scarce further if more than one injection is required, because achieve the same patient return for booster doses make the Immunization Campaign more complicated. A recent uni-dose vaccine reported is the nasal influenza vaccine (F. Martin et al. EP1878424). The World Health Organization includes in its strategic plan for 2006-2009, to develop vaccines that require fewer doses to achieve the desired level of immune response

Other of the main problem of current vaccines is that the vast majority of them are still administered by parenteral route. The infestation by fungi, viruses, bacteria, protozoa and helminths produce a variety of pathologies, common throughout the world. The vast majority of them invades the body through or settles in the mucosal tissue (Brandtzaeg P. and Pabst R. TRENDS in Immunology, 2004.25 (11):570-577). Parenteral immunization is generally effective in clearing systemic infection but it falls to establish protective response at mucosal surface. Nevertheless mucosal immunization stimulates systemic and mucosal immune responses that can prevent pathogen adhesion to host mucosa, tissue invasion, and establishment of disease, leading by the production of secretory immunoglobulin A (SIgA) antibody. The SIgA isotype, is the primary immunoglobulin involved in the protection of mucosal surfaces and is locally produced in the gastrointestinal and upper respiratory tracts, as well as the nose, middle ear, uterine and reproductive mucosa, and glandular tissues

On the other hand based on the compartmentalization of the common mucosal system, other advantage of mucosal immunization is to induce mucosal immunity not only locally at the site of immunization but also to distant sites such as the vagina in the case of intranasal vaccination. Thus, nasal immunization is a preferable route against respiratory pathogens and genital tract, however, is not an option for vaccination against intestinal germs, not to induce responses at this level, which is achieved by the oral route (Brandtzaeg P et al. Immunol Today, 1999, 20:141-151). IgA detected in saliva represents the best marker for measuring the induction of immune response in the nasopharynx and want to find a good marker for oral immunization (Brandtzaeg P. Int. J. Med. Microbiol. 2003,293:3-15).

However, despite the numerous advantages of the mucosal route, very few mucosal vaccines are licensed, only oral vaccines are a fact, although recently a nasal vaccine against influenza, is licensed (FluMist, Belshe RB, Mendelman PM, Treanor J, King J, Gruber WC, Piedra P, et al. NEJM 1998.338: 11405-12).

Either way, the presence of innate mechanisms and the induction of specific IgG systemic are also involved in mucosal protection. So the parenteral route, without inducing SIgA usually protects against infectious diseases, but does not eliminate the carrier stage. The carriers, to carry the germ without usually developing the disease, are of greater epidemiological risk being constant disseminators of infection. Beside many studies have been demonstrated that parenteral route is able to enhance the mucosal immune response induced by mucosal immunization when given in combination as a prime-boost (Giannasca P. J. et al. Infection and Immunity, 1999, 67(2): 527-538; Ranasinghe Ch. et al. Vaccine, 2006, 24: 5881-5895). For that reason is possible combined both rout to enhance the mucosal and systemic immune response.

The high reactogenicity and the unavailability against intracellular pathogens, or infected or altered cells, are others important problems of current vaccines. These problems have been supporting the development of new generation vaccines based on partially purified preparations from the organism or recombinant subunit proteins. However the majorities of new generation vaccines (based on subunits, recombinant proteins or DNA) are likely to be less reactogenics than traditional vaccines but are also less immunogenic, needing a repeated administration of dose. It have been justifies an urgent need for the development of new and non toxics adjuvants for use in human wish also could be administrated by mucosal route.

Adjuvants are substances that enhance specific immune response against an antigen resulting in a faster induction of this and increasing its duration (Vogel FR. Dev. Biol. Stand. 1998,92:241-248). Its use in vaccine formulations can reduce the amount of antigen required, direct the response towards a desired pattern and reduce the number of doses required.

Nevertheless progress in the field of adjuvants for use in human has been inadequate mainly for adjuvants that could be administrated by mucosal rout. Currently, Aluminum compounds (aluminium phosphate and aluminium hydroxide) and MF59 (a squalane o/w emulsion) continue to monopolize human vaccines. Whereas aluminum compounds have been used with a large number of antigens, is not a potent adjuvant that could help to new generation vaccines. Alumin are insufficient in many aspects, including variable adsorption of some antigens, the difficulty to lyophilize, the occurrence of hypersensitivity reactions in some subjects and the inability to be administrated by mucosal rout. Also is considered that only act as delivery system, although recently described a new mechanism of action independent of Toll-like receptors that involves the Nalp3 (Eisenbarth SC et al. Nature, 2008,453:1122-1126) besides that preferentially induce a Th2 response joined the inability to elicit cytotoxic T-cell (CTL) responses.

Adjuvants can be broadly divided into two groups, based on their principal mechanisms of action: Vaccine Delivery Systems and Immunostimulatory adjuvants. Immunostimulatory adjuvants are predominantly derived from pathogens and often represent pathogen associated molecular patterns (PAMP) e.g. LPS, MPL, CpG DNA, which activate cells of the innate immune system. Once activated cells of innate immunity drive and focus the acquired immune response. Vaccine delivery systems are generally particulate (e.g., emulsions, nano and microparticles, immunostimulatory complexes, ISCOMs, liposomes, niosomes and virosomes) and function mainly to target associated antigens into antigen-presenting cells (Pashine A Valianti NM. Nat Med 2005.11: S63-68, Singh M and O 'Hagan DT. Pharm. Res 2002.19 (6):715-728). In addition, (Pérez O. et al. PharmacologyOnline, 2006,3:762-64) consider of extreme importance include in this classification, the immune polarising activity, which re-direct the immune response toward the desired protective response pattern (Immunopolaritation).

Pharmaceutical companies worldwide have focused on complex formulations that combine one or more immunopotentiators with an adequate delivery system. However, licensed vaccine adjuvants are scarce (MF59, AS02, virosomes, AFPL1, AFCo1, Proteollin, MPL, etc.) and few for mucosal application (CTB, LT mutants (LT63K, LTR72), CpG, chitosan, ISCOM and AFCo1) (Singh M, O'Hagan DT. Pharm. Res 2002.19 (6):715-728, Perez O et al. Immunology and Cell Biology. 2004, 82:603-610).

Our group, on the contrary, has focused on getting adjuvants that has multi and synergistic PAMP components (immunopotentiators) that act synergistically, which in turn has delivery capabilities and polarizes the immune response towards Th1 response with preferential induction of CTL responses as well (Perez, et al. Scand J Immunology 2007, 66:271-77, certificate of invention OCPI author of 23,313, 2008 GL Martin Perez et al, WO/2004/047805, WO/2003/094964 and EP1716866)

The term Proteoliposome (PL) was first described by Racker, 1972 name structures which incorporate a membrane protein into a lipid bilayer but it was formerly used to designate liposome-like purified preparations from the outer membrane proteins of *N. meningitidis* (Sierra *et al.,* 1987 and Lowell 1990) which contain high quantity of proteins. PL is also noun as outer membrane vesicle (OMV) a naturally detergent extracted vesicles that have been successfully used for vaccine purposes, the Cuban VA-MENGOC-BC™ (Huergo *et al.* 1997, Sierra *et al.* 1991), the Norwegian (Bjune *et al.,* 1991) and the New Zealand are (Oster *et al.,* 2005) examples of parenteral licensed vaccine against meningococcal B disease. The Cuban meningococcal vaccine, VA-MENGOC-BC™ have been used in more than 60 million doses applied by intramuscular route, with safety and security demonstrated (Sierra *et al.,* 1991). The PL an outer membrane vesicles obtained from live *N. meningitidis* serogroup B strain Cu 385-83 is the core antigen. This PL is also been used as an adjuvants called as AFPL1, (AF 'Adjuvant Finlay' PL1).

Cochleates (Co) are phospholipids-calcium precipitates derived from the interaction of anionic lipid vesicles with divalent cations like calcium. They have a defined multilayered structure consisting of a continuous, solid, lipid bilayer sheet rolled up in a spiral, with hydrophobic internal space. Papahadjopoulos *et al.,* 1975 first described Co as an intermediate in the preparation of large unilamellar liposomes. Since then, these structures have been used to deliver protein, peptide, and DNA for vaccine applications by oral and nasal route (Gould-Fogerite *et al.,* 1998). However, only an antifungal Co delivering Amphotericin B has been licensed, it has shown to be effective, safe and, very stable when administered by oral route (Delmas *et al,* 2002). Therefore, our group developed a novel strategy employs PL from live bacteria as source for lipids, PAMPs, and antigens to induce calcium-cochleate formation (Pérez *et al.* 2005). Then, Adjuvant Finlay Cochleate 1 (AFCo1) is a cochlear structure obtained from AFPL1 by dialysis process, hanging DOC by Ca2+. The interaction of anionic lipid from PL with Ca²⁺, establish an ionic bridge between the two negative charges of lipids from adjacent membranes to stabilize a tubular cochlear microparticles.

The proteoliposomes and derivatives thereof such as cochleates also been used as adjuvants as revealed in a certificate of authorship of 23,313 OCPI invention, Martin GL 2008 Perez et al. and WO/2004/047805.

The Finlay Institute have a platform of adjuvants, the AFPL1 derived from *Neisseria meningitidis* serogroup B and its derivative the Cochleate (Co) (AFCo1) for use in parental and mucosal vaccines including also others PL and Co derivates from other microorganisms.

The AFPL1 and AFCo1 are produced at Finlay Institute under good manufacture practices (GMP). They have exceptional characteristics combining in the same structure, the potentiator activity, polarizing agents and delivery system capacities. Both contain major outer membrane proteins (PorA and PorB), a complex of proteins from 65 to 95 kDa, inserted and controlled amount of lipopolysaccharide, phospholipids and several pathogen-associated molecular pattern (PAMPs) like LPS, Porins, and DNA traces from *N. meningitidis* which are delivered as danger signals to immune competent cells (DC, macrophage, etc.) as well as the antigen incorporated on it (Rodriguez *et al.,* 2005).

The immune response induced by these formulations against its own antigens in murine and human by parenteral administration, is preferential T helper type 1 (Th1) characterized by: IgG subclasses, cytokine (IL) 12, tumor necrosis factor TNFα production of interferon gamma (IFN γ ), try positive delayed hypersensitivity and additionally cytotoxic T lymphocyte (CTL) (Perez O et al. Infect Immun. 2001.69 (7): 4502'-4508, Perez O et al. Immunology and Cell Biology. 2004.82 :603-610, Rodriguez T et al. Vaccine 2005, 26:1312-21, Perez O, et al. Scand J Immunology 2007,66:271-77). The immune response against the antigens included, conjugated or co-administered to their structures is not only potently enhanced but also modulated toward a Th1 pattern too (Pérez *et al*. 2004) administrated by parenteral or mucosal routes (Pérez O et al. Infect Immun. 2001, 69(7):4502-4508; Pérez O, et al. Scand J Immunology 2007,66:271-77). The mucosal AFCo1 action and also AFPL1 applications has been demonstrated by nasal, oral, vaginal and rectal and production has been extended to similar derivatives of other microorganisms. These structures induce an efficient mucosal immune response with specific secretory IgA response on mucosal secretions although AFCo1 induce a significant higher immune response than AFPL1 (Pérez O et al. Immunology and cell Biology. 2004, 82: 603-610)

The chitosan is the most abundant biopolymer in nature after cellulose and is part of the exoskeleton of crustaceans and insects as well as of the cell wall of some microorganisms such as yeasts and fungi. By alkaline deacetylation of chitin N-acetyl-D-glucosamine polymer of different molecular weights and different degrees of deacetylation are obtained which gives it special properties. Due to the lack of toxicity and allergenicity chitosan has numerous applications in the pharmaceutical industry and veterinary medicine.

The chitosan is widely used in research as a transport system for drugs, peptides, proteins, and DNA vaccines, due to its natural mucoadhesive properties. As mucous adhesive polymer, Chitosan has been used to increase the absorption of morphine and insulin through the nasal mucosa epithelium. Chitosan also increase the transepithelial transport of antigens to the mucosal immune tissues through tight junctions and decreased movement mucosiliar as well as induce the immune system given by the effects above macrophages activation and induction of cytokines.

At the level of intestinal epithelium, increases the paracellular transport and the uptake of luminal antigens, this favours the contact with the immune system by increasing the local and systemic immune response. This polysaccharide has also been used as dietary fibber producing changes in intestinal flora in the microenvironment of the mucosa.

Other additional problem of the current vaccines is they not feasibility to combined more than one antigen in the same formulation, to be given at one time and would protect against all major infections disease at the same time, called Combined Vaccines. Combined vaccines allows fewer healthcare, visit, minimizes inconvenience and trauma, mainly for children. Any contact with population for the purpose of vaccination should be an opportunity to administer multiples antigens that protect against multiple infection diseases, particularly in developing countries where population have limited access to medical service. Some Combine Vaccines are available in the Immunization Schedules, some of them combine 3 antigens like DPT and MMR and others combine until 5 like DPT + HepB + Hib. However some of them have gained an unsafe image in the population like MMR to link with the autism in some children that as yet remains unproved scientifically. Others candidates have been showed that the immunological response to one component of the combination was significantly diminished compared with the response to the same antigen inoculated separately. Is also knowing that combined vaccines generally fore better when are administrated mucosally as the vast mucosal surface with its many inductive sites. Likewise concurrent vaccines mean more simultaneous applications of a vaccine antigen, in the same time but in different places. These are based on the regionalization of the immune system that allows inducing different responses in each region, even in the face simultaneous challenges.

**The present invention has as object** the use of PLs (AFPL1) natural or genetically modified bacteria, particularly those derived from *N. meningitidis,* or other microorganism, as well as the Cochleate derivatives from these PL, as novel vaccine adjuvants or vaccines per se when are administered by mucosal and parenteral route at the same time, to obtain unitemporals vaccines.

By "proteoliposomes, PL" means a liposome into which one or more proteins have been inserted, obtained from bacteria using any known method such as: the isolation without detergent, a process that includes detergent (eg deoxycholate, SDS, etc..) or removal from vesicles ( " bleb ") obtained from culture supernatants and in particular those disclosed in U.S. 5,597,572. The PL containing different pathogen-associated molecular patterns (PAMP), molecular structures conserved between pathogens that can strongly stimulate the innate immune system and thereby induce a potent adaptive response. The PL-containing structures of the outer membrane of bacteria, but for the purposes of this patent also considered those extracted from other organisms such as viruses, fungi, protozoa, helminths or tumor cells. The term AFPL is reserved for the use of PL as adjuvant.

By "Cochleate" means a derivative of an PL that also contain several PAMP too as referenced in the patent (WO/2004/047805), Perez O et al. Infect Immun. 2001, 69 (7) :4502-4508 and Perez O et al. Immunology and Cell Biology. 2004, 82:603-610). Also, for the purposes of this patent, Cochleate concept extends to the production of the same from combinations of synthetic lipids with the inclusion of PAMP act synergistically, which differs radically from the other Cochleates produced from lipid synthetic and not included in the patent referred to above.

As "Unitemporals" vaccines means, the simultaneous application of one or more mucosal doses and one parenteral dose, which will achieve effective immunization against one or more vaccine antigens, thus reducing the number of doses, the number of encounters with the person or animal to immunize and thereby increase vaccination coverage by eliminating losses from non-attendance at subsequent doses. This concept extends to any simultaneous combination of antigens adjuvanted or not applied by different routes of immunization at the same time, whether these mucosal or parenteral but preferably the combinations of both route mucosal with parenteral. This concept extends to other vaccines using different adjuvants to the above (proteoliposomes (AFPL) and its derivatives (AFCos). This concept excludes unit dose vaccines which are obviously also applied in a single time.

Most vaccines require two or more doses that generally are applied by the same route of immunization. Therefore, it was surprising to find that the simultaneous application of mucosal and parenteral doses induce higher systemic specific IgG responses than two intramuscular doses or even three mucosal doses.

In our case the use of AFCo1 by intranasal route requires at least three doses to get high nasal mucosal responses, although positive responses are obtained with two doses. Therefore, it has also been surprisingly, found that a single mucosal dose administrated at the same time with a single parenteral dose as unitemporal vaccines, and also induce higher mucosal specific IgA responses than two or even three intranasal dose.

The strategy of stimulation-challenge ('prime-boost'), which emerged to solve the low immunogenicity induced by the promise of naked DNA vaccines and consist in stimulate with interest DNA and subsequently challenged with a different vector expressing the antigen interest or the purified antigen to avoid interference from them. Surprising was also found that only a structural transformation of PL to Cochleate that contains the same PAMP, proteins and phospholipids, enhanced responses achieved by reducing both induce a dose inoculations each way. Additionally it was also surprising that these structures will also work on both tracks in the same unitemporal application. The mucosal and parenteral systems are organized differently and even the mucosal system is more compartmentalized. Due to this compartmentalization, we know that to get immune response in the upper respiratory tract, the intranasal is the best route for protecting against respiratory infections and for protect at digestive tract level only with oral immunizations. Therefore, it was unexpected to find that the simultaneous immunization by either routes, mucosal and parenteral, enhance and further to achieve enhanced responses by the application of formulations by several mucosal routes (nasal, oral, sublingual) at the same time with parenteral routes.

The responses induced by parenteral and mucosal immunization are independent. Both routes usually require several doses and induce cellular response resulting in antibody responses generally different. The parenteral route induces systemic specific IgG responses and the mucosal route, particularly intranasal, induces regional-specific responses of SIgA and also distance (genitourinary tract) as well as systemic specific IgG. Therefore, it was not surprising to find specific systemic IgG responses, since these could be the sum of the responses induced by both routes of immunization, but it was surprising and unexpected that a single nasal dose was boosted by single intramuscular dose, to the level of at least two intranasal doses.

One of the important properties of adjuvants in vaccines is their ability as delivery system, to act as deposits and direct the antigens to the places where T cells are concentrated, to allow immunopotentiators stimulate the innate response. This ensures that antigen releases occur for several days or they may be acquired by antigen-presenting cells for long periods and thus are hauled to T areas of peripheral lymphoid organs, which leads to the latter doses are spaced several weeks apart. Therefore, it was unexpected to observe that similar systemic responses were achieved without spacing between doses. More unexpected still, was found mucosal responses, ensuring that with two applications (one mucosal and one parenteral simultaneously) obtain mucosal and systemic responses. Additionally, was surprised to note that by enhancing the delivery system capacity for the parenteral application with the use of aluminum, chitosan or oil/water emulsions the response unitemporal were also enhanced.

It was also unexpected that the simultaneous application of two or more mucosal routes of two or more antigens and parenteral application in a combined vaccine formulation also induced responses unitemporals efficient. More surprising was finding that these formulations applied unitemporaly; induced memory response which was evidenced by an increased immune response typical of a secondary (memory) response after the application of a mucosal boost several months after the initial immunization.

Also the present invention includes, the obtaining of different vaccine formulations that exploit the ability of the PLs and their derivatives (Cochleates) to induce Th1 responses with CTL activity, using the new strategy.

Poor immunogenic antigen as Bovine Serum Albumin (BSA) or immunogenic such as Tetanus Toxoid (TT), were efficiently evaluated in this unitemporal strategy. These antigens works incorporated or conjugated with the PL and then transformed into Cochleate or co-administered with them.

The vaccine formulations of the present invention can be used for prevention or treatment of infectious diseases of any etiologic and even against tumoral diseases, through unitemporal administration.

Unitemporal administration included as mucosal route: oral, nasal, sublingual, vaginal and rectal and as parenteral routes: intramuscular, intraperitoneal, intradermal, subcutaneous or transcutaneous, always administered in combination at the same time (SinTimVaS).

Generally, the number of doses are two (one mucosal and one parenteral administrated simultaneously at the same time) but also, the invention includes several doses by similar or different mucosal routes with single or combined antigens. Additionally, can be boosted the mucosal and systemic immune response induced by simultaneous similar applications (unitemporals) or only with one of the ways, amplified the memory response initially induced.

The novelty of the invention lies in the unitemporal and simultaneous administration of one or more doses administered by two or more routes of immunization (mucosal and parenteral or mucosal, mucosal and parenteral).

It is particularly new, that unitemporal application of an antigen by mucosal route (oral, sublingual, vaginal, rectal or nasal) and simultaneously, parenteral (subcutaneous, transdermal, intraperitonel, intradermal or intramuscular) induces similar systemic immune response than two parenteral doses spaced 14 days scheme or two mucosal doses spaced 7 days scheme required to induce efficient mucosal and systemic immune responses for both routes.

Another aspect of novelty lies, that unitemporal application of an antigen by mucosal and parenteral routes simultaneously, also induces mucosal immune response that only can be accomplished with at least 2 and better with 3 mucosal doses. To this immune response empowerment there are not current explanation at the level of the T cells driven by signals of cytokines and chemokines.

A final novel aspect is that the Single Time Vaccination Strategy permit that several antigens can be used by one or more mucosal routes and simultaneously the administration of these antigens by parenteral route as a combined vaccine.

The proposed solution has the following advantages:
➢ It extends the concept of stimulation-amplification ('prime-boost') of vaccines, mediating between them weeks or months to induce an amplified secondary response to novel vaccines unitemporals.
➢ It combines the mucosal administration (single or multiple doses) with the parenteral at the same time, resulting in enhanced specific responses of secretory IgA at the mucosal level and IgG systemic.
➢ It uses two or the same adjuvants with similar compositions, instead of two different adjuvants or delivery systems applied in the traditional prime -boost test.
➢ It does increase the low vaccination coverage that exists for non-attendance at subsequent doses of multidose immunization schemes. These results carry on decreasing the necessary resources to conduct immunization campaigns.
➢ It is able to reduce the number of doses in the immunization schedules at least half (two-dose vaccine) or third (three-dose vaccine), without affecting the quality of the systemic immune response, and added the mucosal response, essential for the vast majority of infections occurring particularly in humans.
➢ The effectiveness of AFPL1, one of the proposed adjuvant has been tested in children less than one year, from 2 to 4 years in schoolchildren, adolescents and adults in more than 60 million doses applied by intramuscular route. The other proposed adjuvant AFCo1, derived from the AFPL1, has passed the stability testing and preclinical toxicity for mucosal administration, being a less reactogenic route and does not require sterility, only ensure a controlled microbial load.
➢ Is induced by AFCo1 and AFPL1, specific responses of SIgA, Th1 IgG subclasses with CTL activity when are applied by mucosal or parenteral routes.
➢ The induction of potent responses is given not only against self antigens related to the PLs (homologous) but also against unrelated antigens (heterologous) incorporated, conjugated or co-administered to the structures of these adjuvants and immunized through both mucosal and parenteral route, extending the capabilities of this new unitemporal strategy using these adjuvants as immunopotentiators and delivery systems against multiple vaccine antigens.
➢ The use of multiple antigens by one or more mucosal routes and the application of the corresponding antigens by parenteral route in a combined vaccine permit the development of multiple unitemporals vaccines.

### EXAMPLES

### Example 1. Proteoliposoms Obtention (PL).

The obtention of proteoliposoms began with the culture of the microrganism like *Neisseria meningitidis, Salmonella Typhi, Vibrio cholerae, Echerichia coli, Shiguella* and *Bordetella pertusus* and the biomass collected by centrifugation were subjected to detergent, enzymes and ultrasound extraction process. Cellular debris was removed by centrifugation and the supernatant is then subjected to digestion with nucleases to eliminate nucleic acids. The extract is recovered by ultracentrifugation, resuspended in solution with detergent and purified from other components of low and medium molecular weight by size exclusion chromatography. The proteoliposomes thus obtained contains: porins (PorA and PorB), traces of bacterial DNA and less than 10% (relative to protein) of native LPS inserted into its structure, but never free. Porins, DNA and LPS present are PAMP that interacting with pattern recognition receptors triggering the warning signs at the level of cells involved in the innate response. The final product is subject to a number of biological, physical and chemical controls. In the case of LPS, as well as a PAMP, be the interest antigen it can be increased to at least 35% compared to proteins. Similar procedure for liposomal structures enriched in outer membrane proteins has been used starting from viruses and protozoa.

### Example 2. Obtention of Cochleate derived proteoliposomes

Based on PL obtained by the methods described in EP 5597572 or U.S. 885900077.8, they are resuspended in buffer solution of Tris-EDTA with 0.5% Sodium Deoxycholate. The protein concentration was determined using the modified Lowry method according to Peterson (Analyt. Biochem. 83, 346, 1977) and the content of phospholipids that are incorporated in the PL was determined by quantifying the inorganic phosphorus (Bartlett, J Biol Chem 234, 466, 1959. Both, protein concentration and phospholipid concentration were used to establish the optimal conditions and quantities of detergents needed for the Coclheate formation. We prepared a solution with the PL adjusted to a final concentration of 5-6 mg protein / mL in Tris-EDTA buffer containing sodium deoxycholate in an amount of 6 to 15 times to the total amount of protein, this solution is filtered through a filter with pore size of 0.2µm. Then proceed to make the process of dialysis by tangential filtration or rotational agitation for 24 h with slow continuous change with dialysis buffer Tris 30 mmol/L, NaCl 100 mmol/L and CaCl₂ 5 mmol/L, in H₂O, pH 7.4, under sterile conditions that were preserved during all steps. Coclheate formation was verified by the formation of a white precipitate and subsequent microscopic observation, optical and electronic. The concentration of proteins and phospholipids was re-estimated and adjusted for subsequent trials. The physicochemical properties of proteins included in Cochleate were checked and compared with the PL using polyacrylamide gel electrophoresis stained with Coomassiee Blue. Additionally, the structural integrity of these was checked and verified by Western blot methodology.

### Example 3. The immunization of AFPL1, AFCo1 or VA-MENGOC-BC™ by intramuscular route in mice induce specific anti PL IgG in sera.

**Immunization Schedule:** Female Balb/C mice were immunized with two intramuscular (IM) dose (12.5 µg in 50 µL) with 14-day interval of AFPL1, AFCo1 or VA-MENGOC-BC™.

**Collection method for serum**: Serum samples were obtained 21 days after the last dose. For serum collection animals were bled by retro-orbital puncture and samples were centrifuged 5000g/10 min. The sera were collected, aliquot and frozen at -20°C until the date of the detection of specific IgG antibodies levels by ELISA.

### Detection of specific anti PL IgG in sera by ELISA:

### Reagents:

Buffer Coating (BC): Na₂CO₃ 11 mM, NaHCO₃ 35 mM (pH 9.6)
Phosphate buffered saline (PBS) 0.15 M (pH 7.2)
Blocking solution (BS): PBS, BSA 1%, Tween 20 0,1%
Wash Solution (WS): PBS, Tween 20 0.05% (v/v), pH 7.4
Citrate-Phosphate Buffer (CPB): Na₂HPO₄ 52 mM and citric acid 25 mM (pH 5.6)
Anti-mouse IgG peroxidase-conjugated antibodies (Sigma, St. Louis, MO, EUA)
O-phenylene diamine (OPD) (Sigma, USA)

In-house serum standard curve: Serum obtained from mice with high detectable antibodies responses against *N. meningitidis* serogrup B. Proteoliposome (PL): obtained from live *N. meningitidis* serogroup B strain Cu 385-83 produced at industrial scale under GMP conditions at Finlay Institute, Havana, Cuba

### Method:

- Add 100 µL /well of PL at 20 µg/mL in Buffer Coating and incubate overnight at 4°C using polystyrene 96-well plates (MaxiSorp F96; Nunc, Roskilde, Denmark)
- Add 100 µL /well of blocking solution and incubate for 1 hour at room temperature.
- Add 100 µL /well of the serum samples and serum standard curve at dilution 1:100 in blocking solution and incubate for 2 h at 37°C.
- Add 100 µL /well of Anti-mouse IgG peroxidase-conjugated diluted in Blocking solution at 1/5000 and incubate for 2 h at 37°C.
- Bound antibodies were detected with 100 µL *per* well of the substrate-chromogen mixture (o-phenylenediamine (OPD) and H₂O₂ in citrate-phosphate buffer, pH 5)
- The reaction was stopped by adding 50 µL of H₂SO₄ at 2 mol/L and optical density was measured at 492 nm in a microplate reader (Titertek, Multiskan Plus; Labsystem).
- All incubation steps were followed by three washes with Wash Solution.
- Anti-PL IgG antibodies were expressed in units per mL (U / mL). The means and standard deviation of at least three different experiments are shown.

### Results - expression and calculations

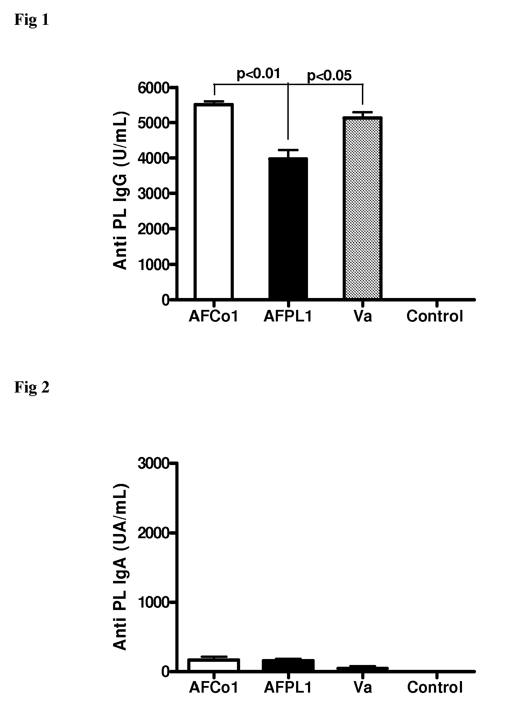

The behaviour parallels between the dilutions of the curve was calculated and thus the linear fit equation and coefficient R². The standard serum was defined as the independent variable (x) and serum samples as the dependent variable (y). Final concentration was determined by substituting in the formula above, the standard values for specific IgG serum, using standard sigmoid curve, assigned 5000 U/mL of IgG anti-PL antibodies to the maximum point on the curve and 31.25 U/mL to the minimum point. The results of IgG anti-PL were calculated by interpolating the OD obtained in each of the serum samples with the calibration curve made with reference serum in U / mL. The detection limit of this assay (less amount of and expressed specific IgG that can be detected under our experimental conditions), was 26.9 U / mL. **Summary:** The adjuvants AFPL1 and AFCo1 immunized by intramuscular route induce similar systemic immune response anti PL than the Cuban antimeningococcal vaccines VA-MENGOC-BC™ although the IgG titters induce by AFCo1 were higher (Fig. 1).

### Example 4. The immunization of AFPL1, AFCo1 or VA-MENGOC-BC™ by intramuscular route in mice not induce specific anti PL secretory IgA in saliva.

**Immunization Schedule:** Female Balb/C mice were immunized with two intramuscular (IM) dose (12.5 µg in 50 µL) of AFPL1, AFCo1 or VA-MENGOC-BC™ with 14-day interval.

**Collection method for saliva:** Saliva was collected at day 7 after the last immunization. Saliva samples were taken following salivation stimulation with intraperitoneal injection of 50 µL of pilocarpine 0.5% (Quimefa, Cuba). Saliva was collected by capillary tube and inactivated 15 min at 56°C to inhibit the protease activity. Then by centrifugation at 10000g/10 min/4°C, the supernatant was collected, aliquoted and frozen at -70°C until the date of the detection of specific IgA antibodies levels by ELISA.

### Detection of specific anti PL IgA in saliva by ELISA:

### Reagents:

Buffer Coating (BC): Na₂CO₃ 11 mM, NaHCO₃ 35 mM (pH 9.6)
Phosphate buffered saline (PBS) 0.15 M (pH 7.2)
Blocking solution (BS): PBS, BSA 1%, Tween 20 0.1%
Washing Solution (WS): PBS, Tween 20 0.05% (v/v), pH 7.4
Citrate-Phosphate buffer (CPB): Na₂HPO₄ 52 mM and citric acid 25 mM (pH 5.6)
Anti-mouse IgA biotinylated antibody (Sigma, St. Louis, MO, EUA)
Streptavidin peroxidase-conjugate (Sigma, St. Louis, MO, EUA)
*O*-phenylene diamine (OPD) (Sigma, USA)

In-house saliva standard curve: Saliva obtains from mice with high detectable antibodies responses against *N. meningitidis* serogrup B.

Proteoliposome (PL): obtained from live *N. meningitidis* serogroup B strain Cu 385-83 produced at industrial scale under GMP conditions at Finlay Institute, Havana, Cuba

### Method:

- Add 100 µL /well of PL at 20µg/mL in Buffer Coating and incubate overnight at 4°C using polystyrene 96-well plates (MaxiSorp F96; Nunc, Roskilde, Denmark)
- Add 100 µL /well of Blocking Solution and incubate for 1 hour at room temperature.
- Add 100 µL /well of the saliva samples and saliva standard curve at dilution 1:2 in blocking solution and incubate for 2 h at 37°C.
- Add 100 µL /well of Anti-mouse IgA biotinylated antibody diluted in blocking solution at 2 µg / mL and incubate for 2 h at 37°C.
- Add 100 µL /well of Streptavidin peroxidase-conjugate diluted in Blocking solution at 1:2000 and incubate for 2 h at 37°C.
- Bound antibodies were detected with 100 µL *per* well of the substrate-chromogen mixture (OPD) and H₂O₂ in citrate-phosphate buffer, pH 5)
- The reaction was stopped by adding 50 µL of H₂SO₄ at 2 mol/L and optical density was measured at 492 nm in a microplate reader (Titertek, Multiskan Plus; Labsystem).
- All incubation steps were followed by three washes with Washing Solution.
- Anti-PL IgA antibodies were expressed in arbitrary units (AU). The means and standard deviation of at least three different experiments are shown.

### Results - expression and calculations

By using standard sigmoid curve, serial twofold dilutions were used and assigned 2000 arbitrary units (AU) of anti PL IgA antibody to the maximum point on the curve and 62.5 AU to the minimum point. The results of IgA anti-PL were calculated by interpolating the OD obtained in each of the samples with the calibration curve made from the saliva of reference and are expressed in AU. The samples were considered positive when values are above capacity of 250 AU, this cut-off value was calculated after evaluating samples of 100 animals and immunized range was established as the average of these samples by 2 standards.

**Summary:** The intramuscular immunization of AFPL1, AFCo1 or VA-MENGOC-BC™ not induces mucosal immune response against PL (Fig. 2).

### Example 5. The intranasal immunization of AFCo1 induces higher mucosal immune response against PL than AFPL1 in mice.

Immunization Schedule: Female Balb/C mice were immunized with three intranasal (IN) dose of AFCo1 or AFPL1 (50 µg in 25 µL, 12.5 µL in each nostril) with 7-day interval

**Collection method for saliva:** Same as Example 4

**Detection of specific anti PL IgA in saliva by ELISA:** Same as Example 4

**Results - expression and calculations:** Same as Example 4

### Summary:

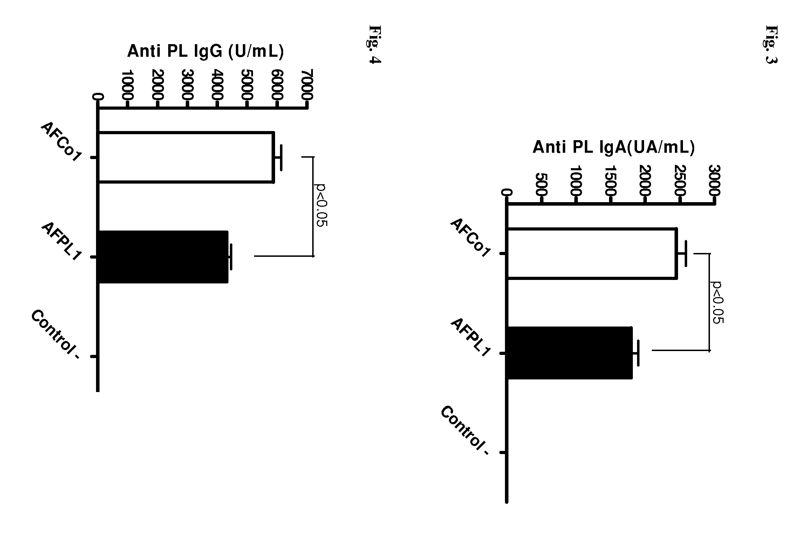

Both formulations AFCo1 and AFPL1 immunized by intranasal route induce specific mucosal immune response against PL being the IgA titters significantly higher with the IN immunization of AFCo1 (Fig. 3).

### Example 6. The intranasal immunization of AFCo1 induces higher systemic immune response against PL than AFPL1 in mice.

**Immunization Schedule:** Female Balb/C mice were immunized with three intranasal (IN) dose of AFCo1 or AFPL1 (50 µg in 25 µL, 12.5 µL in each nostril) with 7-day interval

**Collection method for serum:** Same as Example 3

**Detection of specific anti PL IgG in serum by ELISA:** Same as Example 3

**Results - expression and calculations:** Same as Example 3.

Summary: Both formulations AFCo1 and AFPL1 immunized by intranasal route induce specific systemic immune response against PL being the IgG titters significantly higher with the IN immunization of AFCo1 (Fig. 4).

### Example 7. The intramuscular immunization requires two dose of AFPL1 or AFCo1 to induce an efficient systemic immune response against PL in mice.

**Immunization Schedule:** Female Balb/C mice were immunized with one and two intramuscular (IM) of AFPL1 or AFCo1 (12.5 µg in 50 µL) with 14-day interval.

**Collection method for serum:** Same as Example 3

**Detection of specific anti PL IgG in serum by ELISA:** Same as Example 3

**Results - expression and calculations:** Same as Example 3

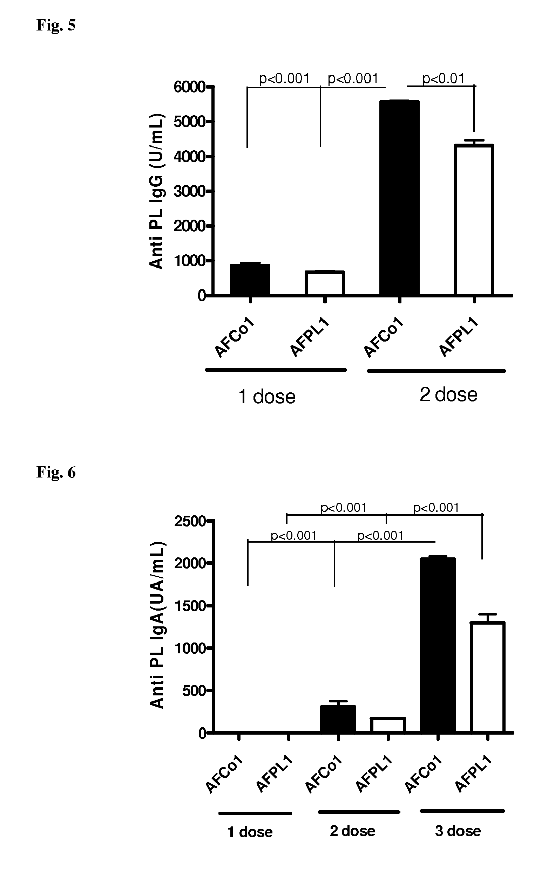

Summary: The AFPL1 and AFCo1 immunized by intramuscular route induce significant IgG titters only when at least two doses are administrated (Fig. 5).

### Example 8. The intranasal immunization requires three dose of AFPL1 or AFCo1 to induce an efficient mucosal immune response against PL in mice.

**Immunization Schedule:** Female Balb/C mice were immunized with one, two and three intranasal dose of AFPL1 or AFCo1 (50 µg in 25 µL, 12.5 µL in each nostril) with 7-day interval.

**Collection method for saliva:** Same as Example 4

**Detection of specific anti PL IgA in saliva by ELISA:** Same as Example 4 **Results - expression and calculations:** Same as Example 4

**Summary:** The AFPL1 and AFCo1 immunized by intranasal route induce mucosal immune response with at least two doses by significant secretory IgA titters only where induce when three doses are administrated (Fig. 6).

### Example 9. The intranasal immunization requires three dose of AFPL1 or AFCo1 to induce an efficient systemic immune response against PL in mice.

**Immunization Schedule:** Female Balb/C mice were immunized with one, two and three intranasal dose of AFPL1 or AFCo1 (50 µg in 25 µL, 12.5 µL in each nostril) with 7-day interval.

**Collection method for serum:** Same as Example 3

**Detection of specific anti PL IgG in serum by ELISA:** Same as Example 3

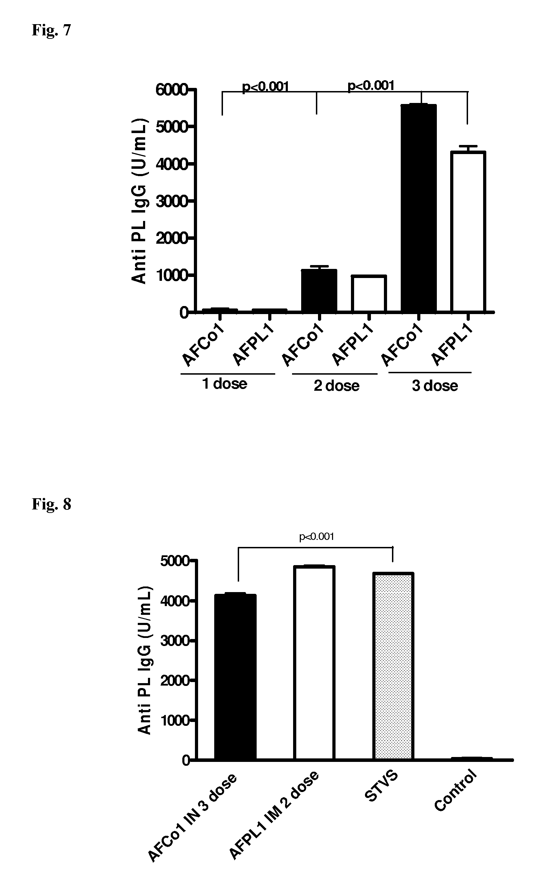

Results - expression and calculations: Same as Example 3 (Fig. 7)

**Summary**: The AFPL1 and AFCo1 immunized by intranasal route induce systemic immune response with at least two doses by significant secretory IgA titters only where induced when three doses are administrated.

### Example 10. The Unitemporal Vaccination of one intranasal dose of AFCo1 and one intramuscular dose of AFPL1 simultaneously induce similar specific IgG responses and same pattern of subclasses than three intranasal doses of AFCo1 and two intramuscular doses of AFPL1, in mice.

**Immunization Schedule:** Balb / C mice were divided into three immunized groups and one control. A first group were immunized with three intranasal doses of AFCo1 (50 µg in 25 µL, 12.5 µL in each nostril) with 7-day interval. A second group were immunized with two intramuscular dose of AFPL1 (12.5 µg in 50 µL per animal) with 14-day interval. The third group was immunized with one intranasal dose of AFCo1 (100 µg in 25 µL, 12.5 µL per nostril) and simultaneously at the same time with one intramuscular dose of AFPL1 (25 µg in 50 µL).

**Collection method for serum:** Same as Example 3

**Detection of specific anti PL IgG in serum by ELISA:** Same as Example 3

### Detection of specific anti PL IgG subclass in serum by ELISA:

### Reagents:

Buffer Coating (BC): Na₂CO₃ 11 mM, NaHCO₃ 35 mM (pH 9.6)
Phosphate buffered saline (PBS) 0.15 M (pH 7.2)
Blocking solution (BS): PBS, BSA 1%, Tween 20 0.1%
Washing Solution (WS): PBS, Tween 20 0.05% (v/v), pH 7.4
Citrate-Phosphate buffer (CPB): Na₂HPO₄ 52 mM and citric acid 25 mM (pH 5.6)
Anti-mouse IgG1, biotinylated whole antibody (from goat) (Amersham, LIFE SCIENCE)
Anti-mouse IgG2a, biotinylated whole antibody (from goat) (Amersham, LIFE SCIENCE)
Streptavidin peroxidase-conjugate (Sigma, St. Louis, MO, EUA)
O-phenylene diamine (OPD) (Sigma, USA)
Proteoliposome (PL): obtained from live *N. meningitidis* serogroup B strain Cu 385-83 produced at industrial scale under GMP conditions at Finlay Institute, Havana, Cuba

### Method:

- Add 100 µL /well of PL at 20µg/mL in Buffer Coating and incubate overnight at 4°C using polystyrene 96-well plates (MaxiSorp F96; Nunc, Roskilde, Denmark)
- Add 100 µL /well of Blocking Solution and incubate for 1 hour at room temperature.
- Add 100 µL /well of the saliva samples and saliva standard curve at dilution 1:2 in blocking solution and incubate for 2 h at 37°C.
- Add 100 µL /well of Anti-mouse IgG1 or IgG2a, biotinylated whole antibody (from goat) diluted in blocking solution at 1:5000 and incubate for 2 h at 37°C.
- Add 100 µL /well of Streptavidin peroxidase-conjugate diluted in Blocking solution at 1:2000 and incubate for 2 h at 37°C.
- Bound antibodies were detected with 100 µL *per* well of the substrate-chromogen mixture (OPD) and H₂O₂ in citrate-phosphate buffer, pH 5)
- The reaction was stopped by adding 50 µL of H₂SO₄ at 2 mol/L and optical density was measured at 492 nm in a microplate reader (Titertek, Multiskan Plus; Labsystem).
- All incubation steps were followed by three washes with Washing Solution.
- Anti-PL IgG subclass antibodies were expressed in optical density units (OD). The means and standard deviation of at least three different experiments are shown.

**Results - expression and calculations:** The results of anti PL IgG1 and IgG2a subclasses were expressed as optical density (OD). In this study samples were considered positive above the 0.25 value of OD.

### Summary:

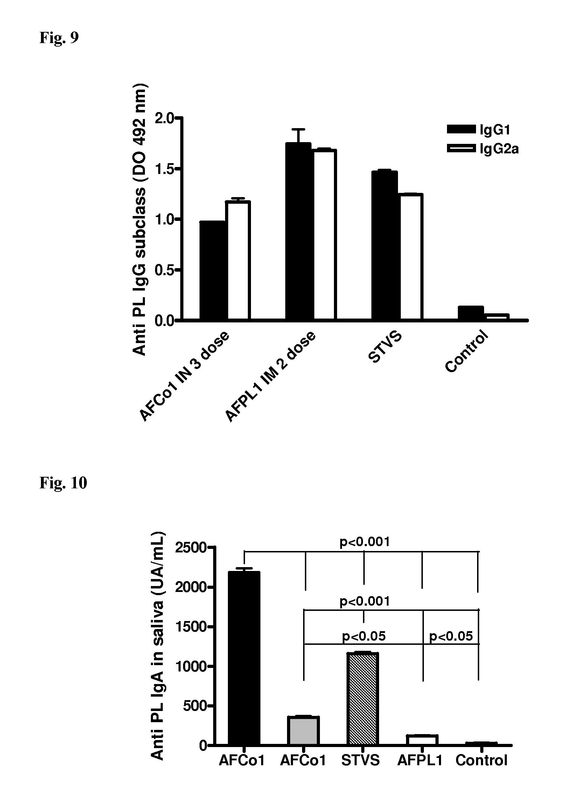

One intranasal dose of AFCo1 and one intramuscular dose of AFPL1 simultaneously as unitemporal strategy induce similar specific IgG responses and same pattern of subclasses than three intranasal doses of AFCo1 and two intramuscular doses of AFPL1, in mice (Fig. 8 and Fig. 9).

### Example 11. The Unitemporal Vaccination of one intranasal dose of AFCo1 and one intramuscular dose of AFPL1 simultaneously induce anti PL IgA antibodies in saliva, faeces and vagina in mice.

**Immunization Schedule:** Balb / C mice were divided into three immunized groups and one control. A first group were immunized with three intranasal doses of AFCo1 (50 µg in 25 µL, 12.5 µL in each nostril) with 7-day interval. A second group were immunized with two intramuscular dose of AFPL1 (12.5 µg in 50 µL per animal) with 14-day interval. The third group was immunized with one intranasal dose of AFCo1 (100 µg in 25 µL, 12.5 µL per nostril) and simultaneously at the same time with one intramuscular dose of AFPL1 (25 µg in 50 µL).

**Collection method for saliva:** Same as Example 4

**Collection method for feces:** 3-5 freshly voided pieces of feces per animal was collected into pre-weighed microassays centrifuge tubes containing homogenization buffer (1 mM PMSF, Aprotinina 5 µg / mL and Leupeptin 1 µg / mL in PBS, pH 7.3) at 20µL/mg of dry feces. Followed by mashing it with a blunt needle, on ice, insoluble material was removed by centrifugation at 14000 rpm / 20 min / 4°C and the supernatants was collected, aliquoted and frozen at -20°C until the date of the detection of specific IgA antibodies levels by ELISA

**Collection method for vaginal secretion:** Vaginal wash samples were obtained by applying approximately 100 µL of sterile PBS into the vagina and aspirating the released fluid. The fluids were centrifuged at 10000g/10 min/4°C and the supernatant was collected, aliquoted and frozen at -20°C until the date of the detection of specific IgA antibodies levels by ELISA.

**Detection of specific anti PL IgA in saliva, feces and vagina by ELISA:** Same as Example 4

### Summary:

One intranasal dose of AFCo1 and one intramuscular dose of AFPL1 simultaneously as Unitemporal Strategy induce specific IgA responses significantly higher than those induced by two intranasal dose of AFCo1 and two intramuscular dose of AFPL1 not only locally at the immunization site but also in places as distant as the vagina and digestive system level (Fig. 10, 11 and 12).

### Example 12. The Unitemporal Vaccination of Ovalbumin using AFCo1 and AFPL1 as mucosal and parenteral adjuvant simultaneously, induce similar anti Ova IgG responses than three intranasal doses of AFCo1+Ova and two intramuscular doses of AFPL1+Ova, in mice.

**Immunization Schedule:** Balb / C mice were divided into four groups immunized and one control. One group was immunized with three IN doses (7 days intervals) of AFCo1+Ova (50 µg / 20 µg in 25 µL per animal, 12.5 µL per nostril). A second group was immunized with two IM doses (14 days intervals) of AFPL1+Ova (12.5 µg / 10 µg in 50 µL per animal). The third group was immunized with one IN dose of AFCo1+Ova (100 µg / 50 µg in 25 µL, 12.5 µL per nostril) and a simultaneous IM dose of AFPL1+Ova (12.5 µg / 10 µg in 50 µL) (STVS). The fourth group was immunized with two IM doses (0, 14 days) of Ova (10 µg in 50 µL per animal).

**Collection method for sera:** Same as Example 3

### Detection of specific anti Ova IgG in sera by ELISA:

### Reagents:

Phosphate buffered saline (PBS) 0.15 M (pH 7.2)
Blocking solution (BS): PBS, BSA 1%, Tween 20 0,1%
Washing Solution (WS): PBS, Tween 20 0,05% (v/v), pH 7.4
Citrate-Phosphate buffer (CPB): Na₂HPO₄ 52 mM and citric acid 25 mM (pH 5.6)
Anti-mouse IgG peroxidase-conjugated antibodies (Sigma, St. Louis, MO, EUA)
O-phenylene diamine (OPD) (Sigma, USA)
Ovalbumine (Ova): Albumin from chicken egg white, Grade V (Sigma, St. Louis, MO, EUA)

### Method:

- Add 100 µL /well of Ova at 10µg/mL in PBS and incubate overnight at 4°C using polystyrene 96-well plates (MaxiSorp F96; Nunc, Roskilde, Denmark)
- Add 100 µL /well of Blocking Solution and incubate for 1 hour at room temperature.
- Add 100 µL /well of the sera samples at dilution 1:100 in blocking solution and incubate for 2 h at 37°C.
- Add 100 µL /well of Anti-mouse IgG peroxidase-conjugated antibodies, diluted in blocking solution at 1:5000 and incubate for 2 h at 37°C.
- Bound antibodies were detected with 100 µL *per* well of the substrate-chromogen mixture (OPD) and H₂O₂ in citrate-phosphate buffer, pH 5)
- The reaction was stopped by adding 50 µL of H₂SO₄ at 2 mol/L and optical density was measured at 492 nm in a microplate reader (Titertek, Multiskan Plus; Labsystem).
- All incubation steps were followed by three washes with Washing Solution.
- Anti-Ova IgG antibodies were expressed in optical density units (OD). The means and standard deviation of at least three different experiments are shown.

### Results: Expression and Calculation

The results of anti-Ova IgG were expressed as optical density (OD). In this study samples were considered positive above the 0.25 value OD.

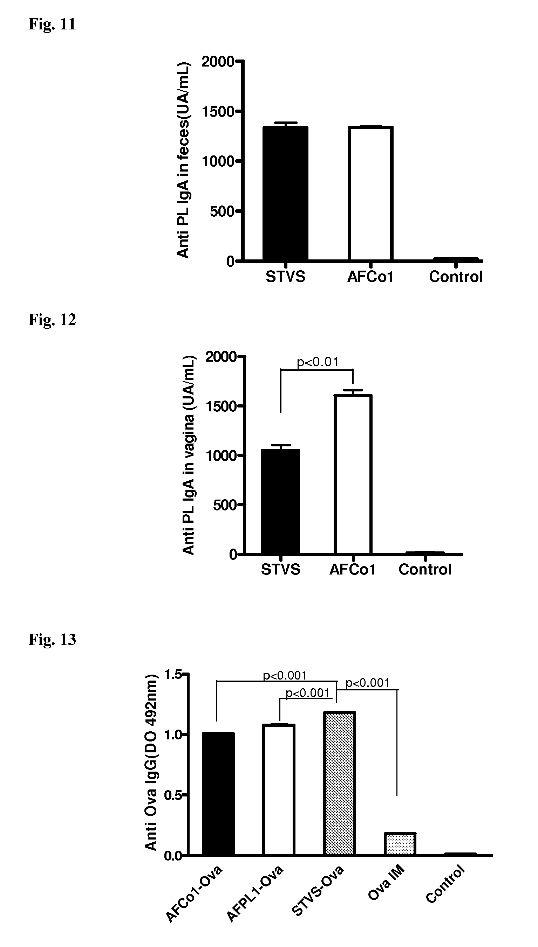

**Summary:** The unitemporal application of Ova using Finlay's Adjuvants induces significant anti-Ova IgG responses in serum. These are superior to those induced by three doses of intranasal AFCo1-Ova and two doses of intramuscular AFPL1-Ova (Fig. 13).

### Example 13. The Unitemporal Vaccination of Ovalbumin using AFCo1 and AFPL1 as mucosal and parenteral adjuvant simultaneously induce in saliva anti Ova IgA responses in mice.

**Immunization Schedule:** Balb / C mice were divided into four groups immunized and one control. One group was immunized with three IN doses (7 days intervals) of AFCo1+Ova (50 µg / 20 µg in 25 µL per animal, 12.5 µL per nostril). A second group was immunized with two IM doses (14 days intervals) of AFPL1+Ova (12.5 µg / 10 µg in 50 µL per animal). The third group was immunized with one IN dose of AFCo1+Ova (100 µg / 50 µg in 25 µL, 12.5 µL per nostril) and simultaneously one IM dose of AFPL1+Ova (12.5 µg / 10 µg in 50 µL) (STVS). The fourth group was immunized with three IN doses of Ova (10 µg in 50 µL per animal).

**Collection method for saliva:** Same as Example 4

**Detection of specific anti Ova IgA in saliva by ELISA:**

### Reagents:

Phosphate buffered saline (PBS) 0.15 M (pH 7.2)
Blocking solution (BS): PBS, BSA 1%, Tween 20 0,1%
Washing Solution (WS): PBS, Tween 20 0,05% (v/v), pH 7.4
Citrate-Phosphate buffer (CPB): Na₂HPO₄ 52 mM and citric acid 25 mM (pH 5.6)
Anti-mouse IgA biotinylated antibody (Sigma, St. Louis, MO, EUA)
Streptavidin peroxidase-conjugate (Sigma, St. Louis, MO, EUA)
O-phenylene diamine (OPD) (Sigma, USA)
Ovalbumine (Ova): Albumin from chicken egg white, Grade V (Sigma, St. Louis, MO, EUA)

### Method:

- Add 100 µL /well of Ova at 10µg/mL in PBS and incubate overnight at 4°C using polystyrene 96-well plates (MaxiSorp F96; Nunc, Roskilde, Denmark)
- Add 100 µL /well of Blocking Solution and incubate for 1 hour at room temperature.
- Add 100 µL /well of the saliva samples at dilution 1:2 in blocking solution and incubate for over night.
- Add 100 µL /well of Anti-mouse IgA biotinylated antibody, diluted in blocking solution at 2 µg / mL and incubate for 2 h at 37°C.
- Add 100 µL /well of Streptavidin peroxidase-conjugate, diluted in blocking solution at 1:2000 and incubate for 2 h at 37°C.
- Bound antibodies were detected with 100 µL *per* well of the substrate-chromogen mixture (OPD) and H₂O₂ in citrate-phosphate buffer, pH 5)
- The reaction was stopped by adding 50 µL of H₂SO₄ at 2 mol/L and optical density was measured at 492 nm in a microplate reader (Titertek, Multiskan Plus; Labsystem).
- All incubation steps were followed by three washes with Washing Solution.
- Anti-Ova IgA antibodies were expressed in optical density units (OD). The means and standard deviation of at least three different experiments are shown.

### Results: Expression and Calculation

The results of anti-Ova IgA were expressed as optical density (OD). In this study samples were considered positive above the 0.25 value OD.

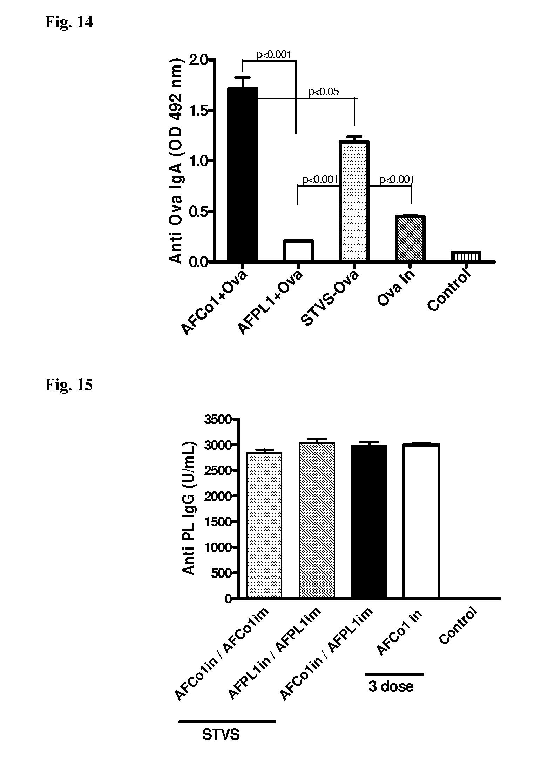

Summary: The unitemporal application of Ova using Finlay's Adjuvants induces positive anti-Ova IgA responses in saliva. These are significantly higher than two intranasal doses of AFCo1-Ova and three intranasal doses of Ova (Fig. 14).

### Example 14. The cochleate (AFCo1) and proteoliposome (AFPL1) can be efficiently used by both routes of immunization (intranasal or intramuscular) in Unitemporal Vaccination Strategy.

**Immunization Schedule:** Balb / C mice were divided into four immunized groups and one control. A first group were immunized with one IN dose of AFCo1 (100µg in 25µL) and one IM doses of AFPL1 (25µg in 50µL) at a single time, the second with one IN dose of AFCo1 (100µg in 25µL) and one IM dose of AFCo1 (25µg in 50µL) at a single time and the third group with one IN dose of AFPL1 (100µg in 25µL) and one IM doses of AFPL1 (25µg in 50µL) at single time too. As positive control group, were immunized with three IN doses (0, 7, 14 days) of AFCo1 (50µg / 25µL per animal 12.5µL for each nostril).

**Method of extraction and processing serum:** proceeded as described in Example 3.

**Detection of anti PL IgG by ELISA:** was proceeded as described in Example 3.

Summary: The AFCo1 and AFPL1 can be efficiently used by both mucosal and parenteral route following the Unitemporal Strategy, inducing efficient systemic and mucosal immune response (Fig. 15).

### Example 15. The cochleate (AFCo1) and proteoliposome (AFPL1) containing Ovalbumin can be efficiently used by both routes of immunization (intranasal or intramuscular) in Unitemporal Vaccination Strategy.

**Immunization Schedule:** Balb / C mice were divided into four immunized groups and one control. A first group were immunized with one IN dose of AFCo1+Ova (100µg / 50µg in 25µL) and one IM doses of AFPL1+Ova (25µg / 20µg in 50µL) at single time, the second group with one IN dose of AFCo1 +Ova (100µg / 50µg in 25µL) and one IM dose of AFCo1+Ova (25µg / 20µg in 50µL) at single time and the third group with one IN dose of AFPL1+Ova (100µg / 50µg in 25µL) and one IM doses of AFPL1+Ova (25µg / 20µg in 50µL) at single time too. As positive control group, were immunized with three IN doses (0, 7, 14 days) of AFCo1+Ova (50µg / 20µg in 25µL per animal 12.5µL for each nostril).

**Method of extraction and processing serum:** proceeded as described in Example 3.

**Detection of anti Ova IgG by ELISA:** was proceeded as described in Example 12.

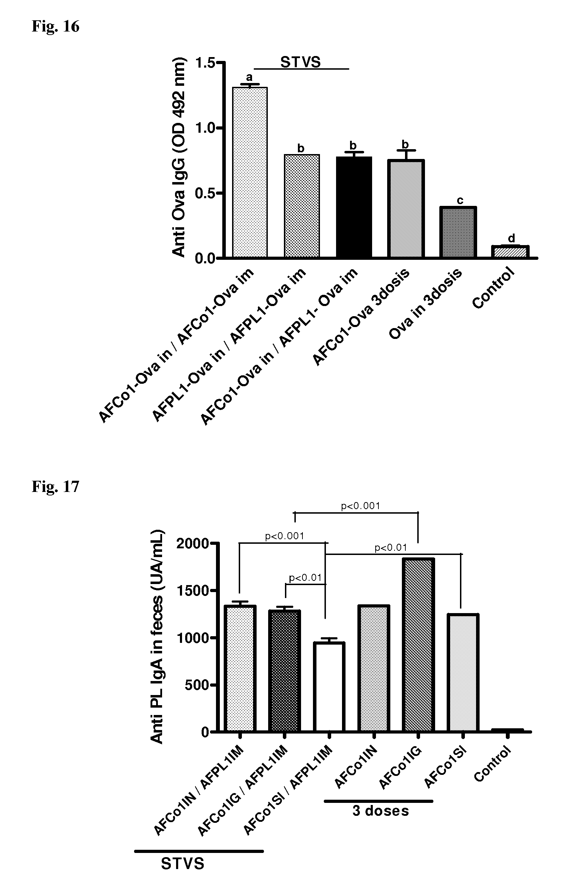

**Summary:** The AFCo1 and AFPL1 can be efficiently used by both mucosal and parenteral route following the Unitemporal Strategy, inducing efficient systemic immune response against the antigen co administered with them (Fig. 16).

### Example 16. In the Unitemporal Vaccination Strategy the administration of single dose of AFCo1 by other mucosal routes (oral or sublingual) simultaneously with a dose of intramuscular AFPL1, induces high levels of specific IgA in saliva, feces and vaginal secretion in mice.

**Immunization Schedule:** Balb / C mice were divided into six immunized groups and one control. The first three groups were immunized with three doses of AFCo1 by intranasal route (50 µg in 25 µL per animal) oral route (100 µg in 200 µL) or sublingual (50 µg in 10 µL per animal), respectively, all doses with an interval of 7 days. The remaining three groups were immunized with one intranasal dose of AFCo1 (100 µg in 25 µL), oral (100 µg in 200 µL) or sublingual (100 µg in 10 µL), respectively and simultaneously one intramuscular dose of AFPL1 (25 µg in 50 µL at the same time.

Collection method for saliva, faeces and vaginal secretion: Same as Example 11

**Detection of specific anti PL IgA in saliva, faeces and vagina by ELISA:** Same as Example 4

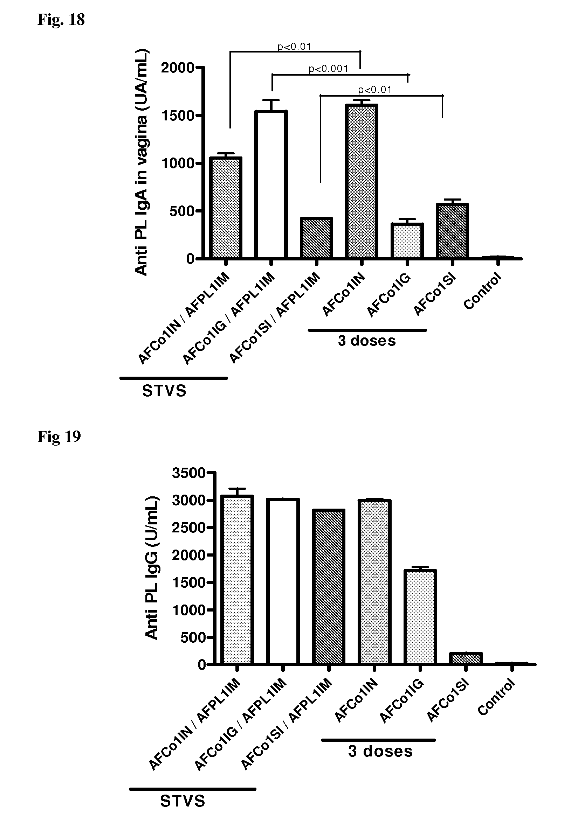

**Summary:** In the unitemporal strategy the administration of one dose of AFCo1 by other mucosal routes (oral or sublingual) simultaneously with one intramuscular dose of AFPL1, induce specific IgA responses significantly higher than those induced by two intranasal dose of AFCo1 and two intramuscular dose of AFPL1 not only locally at the immunization site but also in places as distant as the vagina and digestive system level (Fig. 17 and 18).

### Example 17. In the Unitemporal Vaccination Strategy, a single dose of AFCo1 using other mucosal routes (oral or sublingual) simultaneously with one intramuscular dose of AFPL1, induce similar specific IgG responses than three intranasal doses of AFCo1 and two intramuscular doses of AFPL1, in mice.

**Immunization Schedule:** Balb / C mice were divided into six immunized groups and one control. The first three groups were immunized with three doses of AFCo1 by intranasal route (50 µg in 25 µL per animal) oral route (100 µg in 200 µL) or sublingual (50 µg in 10 µL per animal), respectively, all doses with an interval of 7 days. The remaining three groups were immunized with one intranasal dose of AFCo1 (100 µg in 25 µL), oral (100 µg in 200 µL) or sublingual (100 µg in 10 µL), respectively and simultaneously one intramuscular dose of AFPL1 (25 µg in 50 µL at the same time.

**Collection method for serum:** Same as Example 3

**Detection of specific anti PL IgG in serum by ELISA:** Same as Example 3

**Summary:** In the unitemporal strategy the administration of one dose of AFCo1 by other mucosal routes (oral or sublingual) simultaneously with one intramuscular dose of AFPL1, induce similar specific IgG responses than three intranasal doses of AFCo1 and two intramuscular doses of AFPL1, in mice (Fig. 19)

### Example 18. In the Unitemporal Vaccination Strategy the administration of AFCo1 by mucosal routes combined and simultaneously with one intramuscular dose of AFPL1, induces high levels of specific IgA in feces, in mice.

**Immunization protocol:** Balb/c mice were distributed in six immunised groups and one as control. The first three groups were immunised with three dose of AFCo1 by intranasal rout (50 µg in 25 µL), oral (50 µg in 200 µL) or sublingual (50 µg in 10 µL). The following three groups (4, 5 and 6) were immunised with the unitemporal strategy but combining two mucosal rout with parenteral rout: intranasal / oral /intramuscular, intranasal / sublingual /intramuscular or oral / sublingual / intramuscular respectively. For intranasal rout (100 µg in 25 µL of AFCo1), oral (100 µg in 200 µL of AFCo1) and sublingual (100 µg in 10 µL of AFCo1) simultaneously with one intramuscular dose of AFPL1 (25 µg in 50 µL)

**Method of extraction and processing of stool:** This example performs the extraction of feces as described in Example 11.

**Detection of anti PL IgA by ELISA:** was proceeded as described in Example 4.

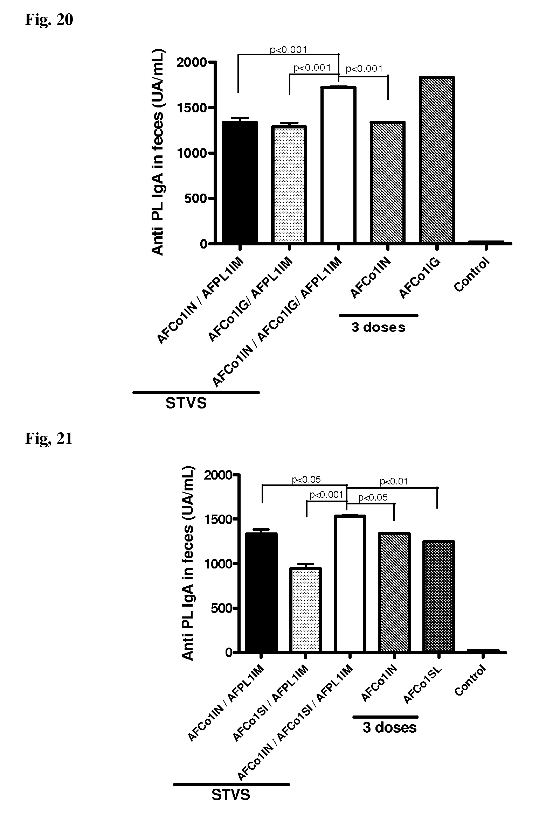

Summary: A Intramuscular dose of AFPL1 at the same time (STVS) with a combined dose of AFCo1 by several mucosal routs, induces mucosal anti PL IgA high levels in feces and vaginal secretion, compared with those induced by three doses of AFCo1 administered intranasally, orally or sublingually (Fig. 20, 21 and 22).

### Example 19: Immunization with tetanus toxoid (TT), diphtheria toxoid (TD) or whole cell pertussis (Pc), co-administered with AFCo1 intranasal, sublingual or oral, respectively, so unitemporal DPT vaccine (adsorbed on alumina ) intramuscularly, induces systemic IgG responses against all antigens.

**Immunization protocol:** Balb / c mice were divided into seven immunized groups and control. First three groups were immunized with AFCo1 + TT (100 mg / 10 LF (flocculation units) in 25 mL) intranasally (IN), AFCo1-TD (100 mg / 25 LF in 25 mL) sublingual (Sl) or AFCo1-P (100 mg / 16 UO (opacity units) in 25 mL) intragastric (IG) respectively. All these groups received simultaneously one intramuscular (IM) dose of DPT vaccines (IM). Groups 4, 5 and 6 were immunized with current schedules of 3 mucosal doses: AFCo1 + TT (IN), AFCo1 + DT (SL) or AFCo1 + P (IG). As control grupo was immunized with two doses of DPT IM.

**Method of extraction and processing of blood to obtain serum**, we proceeded as described in Example 3.

**Extraction and processing method of the saliva** was carried as described in Example 4.

### Determination of IgG anti tetanus toxoid (TT), diphtheria toxoid (DT) and pertussis Cell (PC) by ELISA:

### Reagents:

Tetanus Toxoid: ATPE-Lot 8005 Finlay Institute, c (808LF/mL)
Diphtheria Toxoid: ADPE-Lot 8001 Finlay Institute, c (1200LF/mL)
Whole cell Pertussis (PC): Lot 8006 Finlay Institute, c (756UO/mL)
Blocking solution (BS): PBS, BSA 1%, Tween 20 0,1%
Washing Solution (WS): PBS, Tween 20 0,05% (v/v), pH 7.4
Citrate-Phosphate buffer (CPB): Na₂HPO₄ 52 mM and citric acid 25 mM (pH 5.6)
Anti-mouse IgG peroxidase-conjugated antibodies (Sigma, St. Louis, MO, EUA)
*O*-phenylene diamine (OPD) (Sigma, USA)

### Method:

The methodology was the same for all three antigens, the only variation is in the concentration used for coating
- Add 100 µL /well of TT (10 LF/mL), DT (25 LF/mL) or Pc (16 UO/mL) in PBS and incubate overnight at 4°C using polystyrene 96-well plates (MaxiSorp F96; Nunc, Roskilde, Denmark)
- Add 100 µL /well of Blocking Solution and incubate for 1 hour at room temperature.
- Add 100 µL /well of the sera samples at dilution 1:100 in blocking solution and incubate for 2 h at 37°C.
- Add 100 µL /well of Anti-mouse IgG peroxidase-conjugated antibodies, diluted in blocking solution at 1:5000 and incubate for 2 h at 37°C.
- Bound antibodies were detected with 100 µL *per* well of the substrate-chromogen mixture (OPD) and H₂O₂ in citrate-phosphate buffer, pH 5)
- The reaction was stopped by adding 50 µL of H₂SO₄ at 2 mol/L and optical density was measured at 492 nm in a microplate reader (Titertek, Multiskan Plus; Labsystem).
- All incubation steps were followed by three washes with Washing Solution.
- Anti-TT, DT and Pc IgG antibodies were expressed in optical density units (OD). The means and standard deviation of at least three different experiments are shown.

### Results: Expression and Calculation

The results of anti-Ova IgA were expressed as optical density (OD). In this test, samples were considered positive, above the control value.

**Summary:** We show that the simultaneous application of different antigens, for various mucosal routes, coupled with unitemporal application of the combined vaccine of different antigens (DPT) intramuscularly is effective in inducing immune response. (Figure not shown)

### Example 20. Unitemporal Immunization of AFCo1 Intranasal and AFPL1 intramuscular simultaneously (STVS), induces memory response demonstrated after a booster dose administered at 4 months of immunization.

**Immunization protocol:** Balb / C mice were divided into two groups immunized and one control. One group immunized with two intramuscular doses (0, 14 days) of AFPL1 (12.5 µg in 50 µL per animal) and the second group was immunized with one intranasal dose of AFCo1 (50 µg in 25 µL, 12.5 µL per nostril) and simultaneously (STVS) one intramuscular dose of AFPL1 (12.5 µg in 50 µL). After 120 days (4 months) underwent a challenge with AFCo1 (50 µg in 25 µL, 12.5 µL per nostril) by intranasal route.

**Method of extraction and processing serum:** We proceeded as described in Example 3, adding that the extractions were performed at 21 days, 70, 90 and 120 days after the last dose administered and at 21 days after challenge. **Detection of anti PL IgG by ELISA:** was proceeded as described in Example 3.

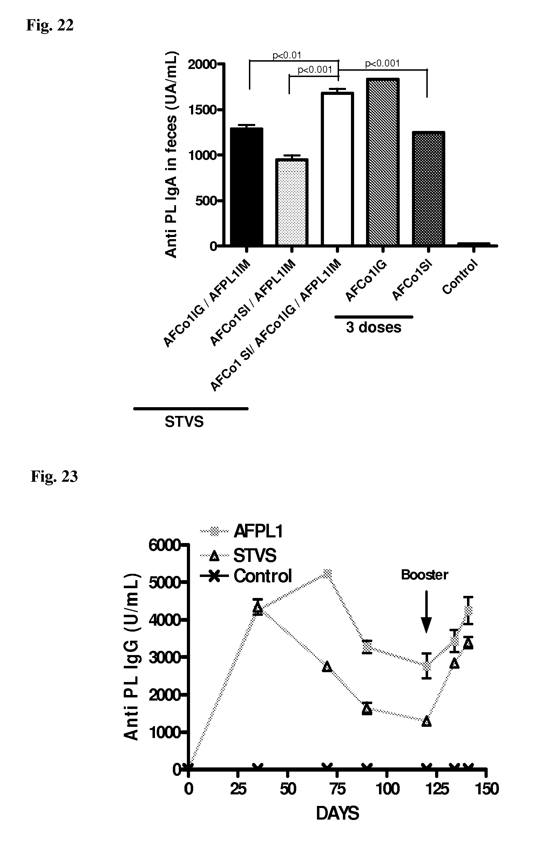

**Summary:** Unitemporal Vaccination Strategy induces not only at effector immune response but also induces appreciated good memory response after administration of a booster dose at 4 months of immunization. (Fig 23)

### Example 21. Immunization with the cochleate containing Ova (AFCo1+Ova) by Intranasal route and simultaneously proteoliposome containing Ova (AFPL1+Ova) by intramuscular rout (STVS) induces anti Ova memory response after a booster dose of OVA administered at 4 months of immunization.

**Immunization protocol:** Balb / C mice were divided into three groups immunized and one control. One group immunized with two intramuscular doses (0, 14 days) of AFPL1+Ova (12.5 µg / 10µg in 50 µL per animal). The second group was immunized with one intranasal dose of AFCo1+Ova (100 µg / 50 µg in 25 µL, 12.5 µL per nostril) and simultaneously (STVS) one intramuscular dose of AFPL1+Ova (25 µg / 20µg in 50 µL). A third group was immunized with two intramuscular doses of Ova (10 µg in 50 µL). After 120 days (4 months) underwent intranasal challenge with AFCo1+Ova (25 µg of Ova in 25 µL, 12.5 µL per nostril) in groups 1 and 2 and intramuscular Ova in group 3.

**Method of extraction and processing serum:** We proceeded as described in Example 3, adding that the extractions were performed at 21 days, 70, 90 and 120 days after the last dose administered and at 21 days after challenge. **Detection of anti Ova IgG by ELISA:** was proceeded as described in Example 12

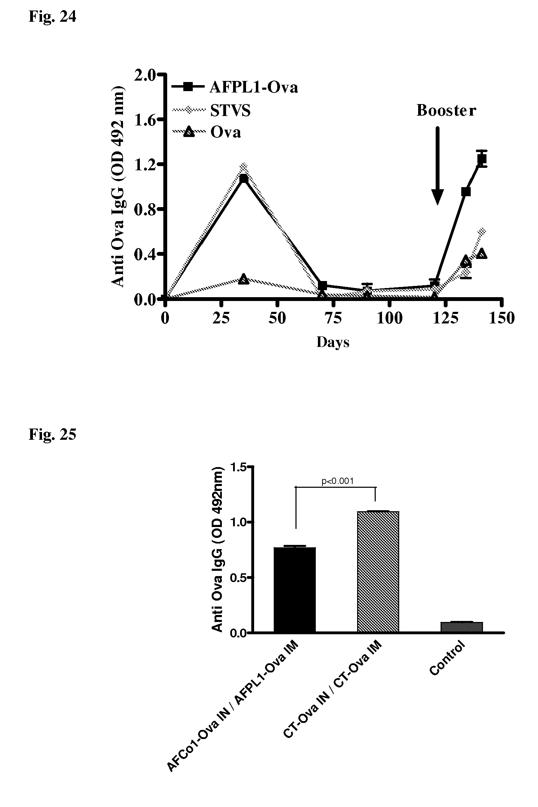

**Summary:** The Unitemporal Vaccination Strategy with OVA as antigen, not only induces effector immune response but at inducing memory response after administer a booster dose of intranasal Ova at 4 months of immunization (Fig 24)

### Example 22: The Unitemporal Immunization works also with other mucosal adjuvants as Cholera Toxin (CT).

**Immunization protocol:** Balb / C mice were divided into three immunized groups and one control. One group was immunized with three intranasal doses (0, 7, 14 days) of CT-Ova (5 µg / 20 µg in 25 µL per animal, 12.5 µL per nostril). A second group was immunized with two intramuscular doses (0, 14 days) of CT-Ova (5 µg /10 µg in 50 µL per animal). The third group was immunized with CT-OVA (10 µg / 20 µg in 20 µL) by intranasal route and simultaneously one intramuscular dose of CT-Ova (5 µg /10 µg in 50 µL).

**Method of extraction serum:** We proceeded as described in Example 3. **Method of extraction saliva, feces and vagina secretion:** We proceeded as described in Example 4

**Detection of anti Ova IgG by ELISA:** We proceeded as described in Example 14.

**Detection of anti Ova IgA by ELISA:** We proceeded as described in Example 15

**Summary:** CT-Ova by intranasal rout applied simultaneously with CT-Ova induced significant systemic and mucosal immune responses against Ova. Therefore, the immunization strategy also works with other unitemporal mucosal adjuvants (Fig. 25, 26 and 27).

### Brief description of the figures

**Figure1****. Anti PL IgG response in sera induced by AFCo1, AFPL1 or VA-MENGOC-BC™ administrated by intramuscular route.** Balb/C mice were immunized with 2 intramuscular doses (0, 14 days) of AFCo1, AFPL1 or VA-MENGOC-BC™ (12.5 µg / 50 µL). For the evaluation of anti PL IgG levels were used serum samples drawn 21 days after the last dose. The determination was made by anti PL IgG ELISA. The figure shows the average and standard deviation of the mathematical relationship of the values (U / mL) of 2 determinations in 3 independent experiments. The different p denote significant differences according to Tukey test (p <0.05).
**Figure 2****. Anti PL IgA response in saliva induced by AFCo1, AFPL1 or VA-MENGOC-BC™ administrated by intramuscular rout.** Balb/C mice were immunised with 2 intramuscular doses (0, 14 days) of AFCo1, AFPL1 or VA-MENGOC-BC™ (12.5 µg / 50 µL). For the evaluation of anti PL IgA levels were used saliva samples extracted 7 days after the last dose immunized. The determination was made by a PL IgA ELISA. The figure shows the average and standard deviation of the mathematical relationship of values (AU / mL) of 2 determinations in 3 independent experiments. The different p denote significant differences according to Tukey test (p <0.05).
**Figure 3****. Anti PL IgA response in saliva induced by AFCo1 or AFPL1 administrated by intranasal route.** Balb/c mice were immunised with three intranasal doses (0, 7, 14 days) of AFCo1 or AFPL1 (50 µg / 25 µL per animal, 12.5 µL through each nostril). For the evaluation of anti PL IgA levels were used saliva samples extracted 7 days after the last dose. The determination was made by a PL IgA ELISA. The figure shows the average and standard deviation of the mathematical relationship of values (AU / mL) of 2 determinations in 3 independent experiments. The different p denote significant differences according to Tukey test (p <0.05).
**Figure 4****. Anti PL IgG response in sera induced by AFCo1 or AFPL1 administrated by intranasal rout.** Balb/C mice were immunised with three intranasal doses (0, 7, 14 days) of AFCo1 or AFPL1 (50 µg / 25 µL per animal, 12.5 µL through each nostril). For the evaluation of anti PL IgG levels were used serum samples drawn 21 days after the last dose. The determination was made by a PL IgG ELISA. The graph shows the average and standard deviation of the mathematical relationship of the values (U / mL) of 2 determinations in 3 independent experiments. The different p denote significant differences according to Tukey test (p <0.05).
**Figure 5****. Anti PL IgG response in sera induced by one and two intramuscular dose of AFCo1 o AFPL1.** Balb/c mice were immunized with one or two intramuscular dose of AFCo1 or AFPL1 (12.5 µg / 50 µL). For the evaluation of anti PL IgG levels were used serum samples drawn 21 days after the last dose. The determination was made by a PL IgG ELISA. The graph shows the average and standard deviation of the mathematical relationship of the values (U / mL) of 2 determinations in 3 independent experiments. The different p denote significant differences according to Tukey test (p <0.05).
**Figure 6****. Anti PL IgA response in saliva induced by one, two and three intranasal dose of AFCo1 or AFPL1.** Balb/c mice were immunised with one, two and three intranasal dose of AFCo1 o AFPL1 (50 µg / 25 µL por animal, 12.5 µL through each nostril). For the evaluation of anti PL IgA levels were used saliva samples extracted 7 days after the last dose. The determination was made by a PL IgA ELISA. The figure shows the average and standard deviation of the mathematical relationship of values (AU / mL) of 2 determinations in 3 independent experiments. The different p denote significant differences according to Tukey test (p <0.05).
**Figure 7****. Anti PL IgG response in sera induced by one, two and three intranasal dose of AFCo1 or AFPL1.** Balb/c mice were immunised with one, two and three intranasal doses of AFCo1 o AFPL1 (50 µg / 25 µL por animal, 12.5 µL through each nostril). For the evaluation of anti PL IgG levels were used serum samples drawn 21 days after the last dose.The determination was made by a PL IgG ELISA. The graph shows the average and standard deviation of the mathematical relationship of the values (U / mL) of 2 determinations in 3 independent experiments. The different p denote significant differences according to Tukey test (p <0.05).
**Figure 8****. Anti PL IgG response in sera induced by the Unitemporal Vaccination (STVS) of AFCo1 and AFPL1 by intransal and intramuscular rout respectively.** Balb/c mice were were distributed in three groups immunized and one as control. The first group was immunised with three intranasal doses (0, 7, 14 days) of AFCo1 (50 µg / 25 µL per animal, 12.5 µL through each nostril). The second group was immunised with two intramuscular doses (0, 14 days) of AFPL1 (12.5 µg / 50 µL per animal). The third group was immunised with one intranasal dose of AFCo1 (100 µg / 25 µL per animal, 12.5 µL through each nostril) and simultaneously one intramuscular dose of AFPL1 (25 µg / 50 µL)(STVS). For the evaluation of anti PL IgG levels were used serum samples drawn 21 days after the last dose. The determination was made by a PL IgG ELISA. The figure shows the average and standard deviation of the mathematical relationship of the values (U / mL) of 2 determinations in 3 independent experiments. The different p denote significant differences according to Tukey test (p <0.05).
**Figure 9****. Anti PL IgG1 and IgG2a response in sera induced by the Unitemporal Vaccination (STVS) of AFCo1 and AFPL1 by intransal and intramuscular rout respectively.** Balb/C mice were distributed in three groups immunized and one as control. The first group was immunised with three intranasal doses (0, 7, 14 days) of AFCo1 (50 µg / 25 µL per animal, 12.5 µL through each nostril). The second group was immunised with two intramuscular doses (0, 14 days) of AFPL1 (12.5 µg / 50 µL per animal). The third group was immunised with one intranasal dose of AFCo1 (100 µg / 25 µL per animal, 12.5 µL through each nostril) and simultaneously one intramuscular dose of AFPL1 (25 µg / 50 µL)(STVS). For the evaluation of the levels of IgG subclasses of PL were used serum samples drawn 21 days after the last dose. The determination was made by ELISA of IgG subclasses of PL. The figure shows the average and standard deviation of the mathematical relationship of the values of optical density (OD) of 2 determinations in 3 independent experiments for each formulation.
**Figure 10****. Anti PL IgA response in saliva induced by the Unitemporal Vaccination (STVS) of AFCo1 and AFPL1 by intransal and intramuscular rout respectively.** Balb/c mice were distributed in three groups immunized and one as control. The first group was immunised with three intranasal doses (0, 7, 14 days) of AFCo1 (50 µg / 25 µL per animal, 12.5 µL through each nostril). The second group was immunised with two intramuscular doses (0, 14 days) of AFPL1 (12.5 µg / 50 µL per animal). The third group was immunised with one intranasal dose of AFCo1 (100 µg / 25 µL per animal, 12.5 µL through each nostril) and simultaneously one intramuscular dose of AFPL1 (25 µg / 50 µL) (STVS). For the evaluation of the levels of anti Pl IgA antibodies were used saliva drawn 7 days after the last dose. The determination was made by a PL IgA ELISA. The figure shows the average and standard deviation of the mathematical relationship of values (AU / mL) of 2 determinations in 3 independent experiments. A P-value of <0.05 was considered statistically significant and it is represent by different letters, a (p<0.001); b is (p<0.05) and c (p <0.01) according to Tukey test.
**Figure 11****. Anti PL IgA response in feces induced by the Unitemporal Vaccination (STVS) of AFCo1 and AFPL1 by intransal and intramuscular rout respectively.** Balb/c mice were distributed in three groups immunized and one as control. The first group was immunised with three intranasal doses (0, 7, 14 days) of AFCo1 (50 µg / 25 µL per animal, 12.5 µL through each nostril). The second group was immunised with two intramuscular doses (0, 14 days) of AFPL1 (12.5 µg / 50 µL per animal). The third group was immunised with one intranasal dose of AFCo1 (100 µg / 25 µL per animal, 12.5 µL through each nostril) and simultaneously one intramuscular dose of AFPL1 (25 µg / 50 µL) (STVS). For the evaluation of the levels of anti Pl IgA antibodies were used feces samples drawn 14 days after the last dose. The determination was made by a PL IgA ELISA. The figure shows the average and standard deviation of the mathematical relationship of values (AU / mL) of 2 determinations in 3 independent experiments. A P-value of <0.05 was considered statistically significant and it is represent by different letters, a (p<0.001); b is (p<0.05) and c (p <0.01) according to Tukey test.
**Figure 12****. Anti PL IgA response vaginal secretion induced by the Unitemporal Vaccination (STVS) of AFCo1 and AFPL1 by intransal and intramuscular rout respectively.** Balb/c mice were distributed in three groups immunized and one as control. The first group was immunised with three intranasal doses (0, 7, 14 days) of AFCo1 (50 µg / 25 µL per animal, 12.5 µL through each nostril). The second group was immunised with two intramuscular doses (0, 14 days) of AFPL1 (12.5 µg / 50 µL per animal). The third group was immunised with one intranasal dose of AFCo1 (100 µg / 25 µL per animal, 12.5 µL through each nostril) and simultaneously one intramuscular dose of AFPL1 (25 µg / 50 µL) (STVS). For the evaluation of the levels of anti Pl IgA antibodies were used vaginal secretion obtained 21 days alter the last dose. The determination was made by a PL IgA ELISA. The figure shows the average and standard deviation of the mathematical relationship of values (AU / mL) of 2 determinations in 3 independent experiments. A P-value of <0.05 was considered statistically significant and it is represent by different letters, a (p<0.001); b is (p<0.05) and c (p <0.01) according to Tukey test.
**Figure 13****. Anti Ova IgG response in sera induced by the unitemporal Vaccination of AFCo1+Ova Intranasal and AFPL1+Ova Intramuscular.** Balb/C mice were distributed in four groups immunized and one as control. The first group was immunised with three intranasal doses (0, 7, 14 days) of AFCo1+Ova (50 µg / 25 µg in 25 µL per animal, 12.5 µL through each nostril). The second group was immunised with two intramuscular doses (0, 14 days) of AFPL1+Ova (12.5 µg / 10 µg in 50 µL per animal). The third group was immunised with one intranasal dose of AFCo1+Ova (100 µg / 50 µg in 25 µL per animal, 12.5 µL through each nostril) and simultaneously one intramuscular dose of AFPL1+Ova (25 µg / 20 µg in 50 µL) (STVS). The fourth group was immunised with two intramuscular doses (0, 14 days) of Ova (10 µg in 50 µL per animal) as antigen control without adjuvant. For the evaluation of anti-OVA IgG levels were used serum samples drawn 21 days after the last dose. The determination was made by Ova IgG ELISA. The figure shows the average and standard deviation of the mathematical relationship of values (OD) of 2 determinations in 3 independent experiments. The different p denote significant differences according to Tukey test (p <0.05).
**Figure 14****. Anti Ova IgA response in saliva induced by the Unitemporal Vaccination (STVS) of AFCo1 Intranasal and AFPL1 Intramuscular containing Ovalbumin as model antigen.** Balb/C mice were distributed in four groups immunized and one as control. The first group was immunised with three intranasal doses (0, 7, 14 days) of AFCo1+Ova (50 µg / 25 µg in 25 µL per animal, 12.5 µL through each nostril). The second group was immunised with two intramuscular doses (0, 14 days) of AFPL1+Ova (12.5 µg / 10 µg in 50 µL per animal). The third group was immunised with one intranasal dose of AFCo1+Ova (100 µg / 50 µg in 25 µL per animal, 12.5 µL through each nostril) and simultaneously one intramuscular dose of AFPL1+Ova (25 µg / 20 µg in 50 µL) (STVS). The fourth group was immunised with three intranasal doses (0, 7 and 14 days) of Ova (20 µg in 25 µL per animal, 12.5 µL through each nostril) as antigen control without adjuvant. For the evaluation of anti-OVA IgA levels were used saliva samples drawn 7 days after the last dose. The determination was made by Ova IgA ELISA. The figure shows the average and standard deviation of the mathematical relationship of optical density values (OD) of 2 determinations in 3 independent experiments. The different p denote significant differences according to Tukey test (p <0.05).
**Figure 15****. Anti PL IgG response induced by the Unitemporal Vaccination of AFCo1 Intranasal and Intramuscular simultaneously or AFPL1 Intranasal and Intramuscular simultaneously too (STVS homologous).** Balb/C mice were distributed in four groups immunized and one as control. The first group was immunised with one intranasal (100 µg in 25 µL per animal, 12.5 µL through each nostril) and one intramuscular dose (25 µg in 50 µL per animal) simultaneously of AFCo1. The second group was immunised in the same manner but using AFPL1. The third group was immunised using the same Schedule but using AFCo1 and AFPL1 by intranasal and intramuscular route respectively. As positive control group (fourth) the animals were immunised with three intranasal doses (0, 7 and 14 days) of AFCo1 (50 µg in 25 µL per animal, 12.5 µL through each nostril). For the evaluation of anti PL IgG levels were used serum samples drawn 21 days after the last dose. The determination was made by a PL IgG ELISA. The figure shows the average and standard deviation of the mathematical relationship of the values (U / mL) of 2 determinations in 3 independent experiments. The different p denote significant differences according to Tukey test (p <0.05).
**Figure 16****. Anti Ova IgG response induced by the Unitemporal Vaccination of AFCo1 Intranasal and Intramuscular simultaneously or AFPL1 Intranasal and Intramuscular simultaneously too, containing Ovalbumin as model antigen (STVS homologous).** Balb/C mice were distributed in four groups immunized and one as control. The first group was immunised with one intranasal (100 µg / 50 µg in 25 µL per animal, 12.5 µL through each nostril) and one intramuscular dose (25 µg / 20 µg in 50 µL per animal) simultaneously of AFCo1+Ova. The second group was immunised in the same manner but using AFPL1+Ova. The third group was immunised using the same Schedule but using AFCo1+Ova and AFPL1+Ova by intranasal and intramuscular route respectively. As positive control group (fourth and fifth) the animals were immunised with three intranasal dose (0, 7 and 14 days) of AFCo1 + Ova (50 µg in 25 µL per animal, 12.5 µL through each nostril) and Ova (25 µg in 25 µL per animal, 12.5 µL through each nostril) respectively. For the evaluation of anti Ova IgG levels were used serum samples drawn 21 days after the last dose. The determination was made by a Ova IgG ELISA. The figure shows the average and standard deviation of the mathematical relationship of optical density values (OD) of 2 determinations in 3 independent experiments. A P-value of <0.05 was considered statistically significant and it is represent by different letters, a (p<0.001); b is (p<0.05) and c (p <0.01) according to Tukey test.
**Figure 17****. Anti PL IgA response in feces induced by Unitemporal Vaccination of AFCo1 using other mucosal rout (oral or sublingual) and AFPL1 by intramuscular rout.** Balb/C mice were distribute in six immunised groups and one as control the first three groups were immunised with three dose of AFCo1 by intranasal rout (50 µg in 25 µL), oral (50 µg in 200 µL) or sublingual (50 µg in 10 µL). The following three groups (4, 5 and 6) were immunised with the unitemporal strategy, one mucosal dose of AFCo1, intranasal rout (100 µg in 25 µL), oral (100 µg in 200 µL) or sublingual (100 µg in 10 µL) respectively and simultaneously one intramuscular dose of AFPL1 (25 µg in 50 µL). For the evaluation of the levels of anti PL IgA antibodies were used feces samples drawn 14 days after the last dose. The determination was made by a PL IgA ELISA. The figure shows the average and standard deviation of the mathematical relationship of values (AU / mL) of 2 determinations in 3 independent experiments. A P-value of <0.05 was considered statistically significant and it is represent by different letters, a (p<0.001); b is (p<0.05) and c (p <0.01) according to Tukey test.
**Figure 18****. Anti PL IgA response in vaginal secretion induced by the Unitemporal Vaccination of AFCo1 using other mucosal rout (oral or sublingual) with AFPL1 by intramuscular rout.** Balb/C mice were distribute in six immunised groups and one as control the first three groups were immunised with three dose of AFCo1 by intranasal rout (50 µg in 25 µL), oral (50 µg in 200 µL) or sublingual (50 µg in 10 µL). The following three groups (4, 5 and 6) were immunised with the unitemporal strategy, one mucosal dose of AFCo1, intranasal rout (100 µg in 25 µL), oral (100 µg in 200 µL) or sublingual (100 µg in 10 µL) respectively and simultaneously one intramuscular dose of AFPL1 (25 µg in 50 µL). For the evaluation of the levels of anti PL IgA antibodies were used vaginal secretion obtained 21 days alter the last dose. The determination was made by a PL IgA ELISA. The figure shows the average and standard deviation of the mathematical relationship of values (AU / mL) of 2 determinations in 3 independent experiments. A P-value of <0.05 was considered statistically significant and it is represent by different letters, a (p<0.001); b is (p<0.05) and c (p <0.01) according to Tukey test.
**Figure 19****. Anti PL IgG response in sera induced by the Unitemporal Vaccination of AFCo1 using other mucosal rout (oral or sublingual) with AFPL1 by intramuscular rout.** Balb/C mice were distributed in six immunised groups and one as control. The first three groups were immunised with three dose of AFCo1 by intranasal rout (50 µg in 25 µL), oral (50 µg in 200 µL) or sublingual (50 µg in 10 µL). The following three groups (4, 5 and 6) were immunised with the unitemporal strategy, one mucosal dose of AFCo1, intranasal rout (100 µg in 25 µL), oral (100 µg in 200 µL) or sublingual (100 µg in 10 µL) respectively and simultaneously one intramuscular dose of AFPL1 (25 µg in 50 µL). For the evaluation of the levels of anti PL IgG antibodies were used SERA drawn 21 days after the last dose. The determination was made by a PL IgG ELISA. The figure shows the average and standard deviation of the mathematical relationship of values (U / mL) of 2 determinations in 3 independent experiments. A P-value of <0.05 was considered statistically significant and it is represent by different letters, a (p<0.001); b is (p<0.05) and c (p <0.01) according to Tukey test.
**Figure 20****. Anti PL IgA response in feces induced by Unitemporal Vaccination of AFCo1 by intranasal and oral rout combined, simultaneously with intramuscular dose of AFPL1.** Balb/C mice were distributed in five immunised groups and one as control. The first two groups were immunised with three dose of AFCo1 by intranasal rout (50 µg in 25 µL) and by oral rout (50 µg in 200 µL) respectively. The following two groups were immunised with the unitemporal strategy but combining two mucosal rout intranasal / oral with parenteral rout, using the following doses: for intranasal (100 µg in 25 µL of AFCo1), oral (100 µg in 200 µL of AFCo1) and for intramuscular (25 µg in 50 µL of AFPL1). For the evaluation of the levels of anti PL IgA antibodies were used feces samples drawn 14 days after the last dose. The determination was made by a PL IgA ELISA. The figure shows the average and standard deviation of the mathematical relationship of values (UA / mL) of 2 determinations in 3 independent experiments. A P-value of <0.05 was considered statistically significant and it is represent by different letters, a (p<0.001); b is (p<0.05) and c (p <0.01) according to Tukey test.
**Figure 21****. Anti PL IgA response in feces induced by Unitemporal Vaccination of AFCo1 by intranasal and sublingual rout combined, simultaneously with intramuscular dose of AFPL1.** Balb/C mice were distributed in five immunised groups and one as control. The first two groups were immunised with three dose of AFCo1 by intranasal rout (50 µg in 25 µL) and by sublingual rout (50 µg in 25 µL) respectively. The following two groups were immunised with the unitemporal strategy but combining two mucosal rout intranasal / sublingual with parenteral rout, using the following doses: for intranasal and sublingual (100 µg in 25 µL of AFCo1) and for intramuscular (25 µg in 50 µL of AFPL1). For the evaluation of the levels of anti PL IgA antibodies were used feces samples drawn 14 days after the last dose. The determination was made by a PL IgA ELISA. The figure shows the average and standard deviation of the mathematical relationship of values (UA / mL) of 2 determinations in 3 independent experiments. A P-value of <0.05 was considered statistically significant and it is represent by different letters, a (p<0.001); b is (p<0.05) and c (p <0.01) according to Tukey test.
**Figure 22****. Anti PL IgA response in feces induced by Unitemporal Vaccination of AFCo1 by oral and sublingual rout combined, simultaneously with intramuscular dose of AFPL1.** Balb/C mice were distributed in five immunised groups and one as control. The first two groups were immunised with three dose of AFCo1 by oral rout (50 µg in 100 µL) and by sublingual rout (50 µg in 25 µL) respectively. The following two groups were immunised with the unitemporal strategy but combining two mucosal rout oral / sublingual with parenteral rout, using the following doses: for sublingual (100 µg in 25 µL of AFCo1), for oral rout oral (100 µg in 200 µL of AFCo1) and for intramuscular (25 µg in 50 µL of AFPL1). For the evaluation of the levels of anti PL IgA antibodies were used feces samples drawn 14 days after the last dose. The determination was made by a PL IgA ELISA. The figure shows the average and standard deviation of the mathematical relationship of values (UA / mL) of 2 determinations in 3 independent experiments. A P-value of <0.05 was considered statistically significant and it is represent by different letters, a (p<0.001); b is (p<0.05) and c (p <0.01) according to Tukey test.
**Figure 23****. Anti PL memory immune response induced by the Unitemporal Vaccination of AFCo1 Intranasal and AFPL1 intramuscular (STVS).** Balb/C mice were distributed in two immunised groups and one as control. The first group was immunised with two intramuscular doses (0, 14 days) of AFPL1 (12.5 µg in 50 µL per animal). The second group was immunised with the new strategy: one intranasal dose of AFCo1 (100 µg in 25 µL, 12.5 µL through each nostril) and simultaneously one intramuscular dose of AFPL1 (25 µg in 50 µL). After 120 days (4 months) a challenge was done with 50µg of AFCo1 intranasally in 25 µL, 12.5 µL per nostril. For the evaluation of anti PL IgG levels were used serum samples drawn 21, 70, 90 and 120 days after the last dose of immunization and at 14 and 21 days after challenge. The determination was made by a PL IgG ELISA. The figure shows the average and standard deviation of the mathematical relationship of values (U / mL) of 2 determinations in 3 independent experiments. The different p denote significant differences according to Tukey test (p <0.05).
**Figure 24****. Anti Ova memory immune response induced by the Unitemporal Vaccination of AFCo1 Intranasal and AFPL1 intramuscular with Ovalbumin as model antigen (STVS).** Balb/C mice were distributed in three immunised groups and one as control. The first group was immunised with two intramuscular doses (0, 14 days) of AFPL1+Ova (12.5 µg / 10 µg in 50 µL per animal) and the second with two intramuscular doses (0, 14 days) of Ova (10 µg in 50 µL per animal). The third group was immunised with the new strategy: one intranasal dose of AFCo1+Ova (100 µg / 50 µg in 25 µL, 12.5 µL through each nostril) and simultaneously one intramuscular dose of AFPL1+Ova (25 µg / 20 µg in 50 µL). After 120 days (4 months) a challenge was done with AFCo1 +Ova (50µg / 20 µg in 25 µL, 12.5 µL per nostril) by intranasal rout. For the evaluation of anti Ova IgG levels were used serum samples drawn 21, 70, 90 and 120 days after the last dose of immunization and at 14 and 21 days after challenge. The determination was made by an Ova IgG ELISA. The figure shows the average and standard deviation of the mathematical relationship of values (OD) of 2 determinations in 3 independent experiments. The different p denote significant differences according to Tukey test (p <0.05).
Figure 25. Anti Ova IgG response in sera induced by the unitemporal Vaccination using other mucosal adjuvant as Cholera Toxin (CT). Balb/c mice were were distributed in three groups immunized and one as control. The first group was immunised with one intranasal dose of CT+Ova (5 µg / 50 µg in 25 µL per animal, 12.5 µL through each nostril) and simultaneously one intramuscular dose of CT+Ova (5 µg / 20 µg in 50 µL) (STVS). The fourth and fifth group was immunised with two intramuscular doses (0, 14 days) of Ova (10 µg in 50 µL per animal) and three intranasal dose (0, 7 and 14 days) of Ova (20 µg in 25 µL per animal, 12.5 µL through each nostril) as antigen control without adjuvant. For the evaluation of anti-OVA IgG levels were used serum samples drawn 21 days after the last dose. The determination was made by Ova IgG ELISA. The figure shows the average and standard deviation of the mathematical relationship of values (OD) of 2 determinations in 3 independent experiments. The different p denote significant differences according to Tukey test (p <0.05).

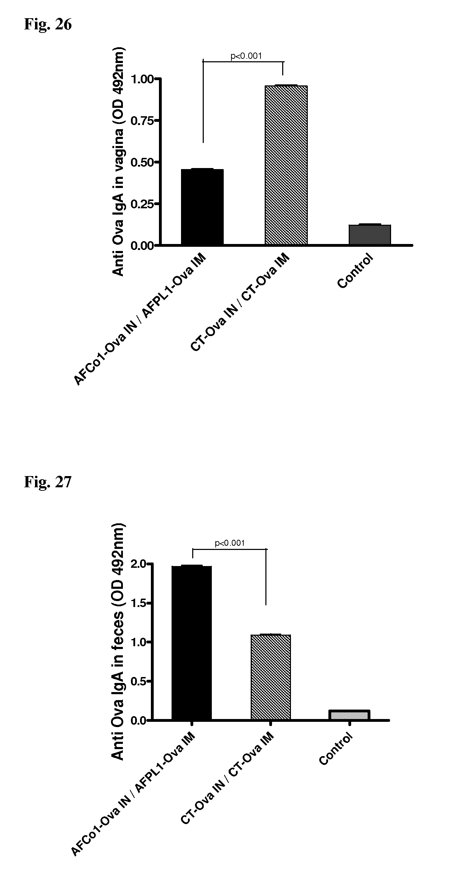

**Figure 26****. Anti Ova IgA response in vagina induced by the Unitemporal Vaccination (STVS) of AFCo1 Intranasal and AFPL1 Intramuscular containing Ovalbumin as model antigen.** Balb/C mice were distributed in three groups immunized and one as control. The first group was immunised with one intranasal dose of CT+Ova (5 µg / 50 µg in 25 µL per animal, 12.5 µL through each nostril) and simultaneously one intramuscular dose of CT+Ova (5 µg / 20 µg in 50 µL) (STVS). The fourth and fifth group was immunised with two intramuscular doses (0, 14 days) of Ova (10 µg in 50 µL per animal) and three intranasal dose (0, 7 and 14 days) of Ova (20 µg in 25 µL per animal, 12.5 µL through each nostril) as antigen control without adjuvant For the evaluation of anti-OVA IgA levels were used vaginal wash samples drawn 21 days after the last dose. The determination was made by Ova IgA ELISA. The figure shows the average and standard deviation of the mathematical relationship of optical density values (OD) of 2 determinations in 3 independent experiments. The different p denote significant differences according to Tukey test (p <0.05).
**Figure 27****. Anti Ova IgA response in feces induced by the Unitemporal Vaccination (STVS) of AFCo1 Intranasal and AFPL1 Intramuscular containing Ovalbumin as model antigen.** Balb/C mice were distributed in three groups immunized and one as control. The first group was immunised with one intranasal dose of CT+Ova (5 µg / 50 µg in 25 µL per animal, 12.5 µL through each nostril) and simultaneously one intramuscular dose of CT+Ova (5 µg / 20 µg in 50 µL) (STVS). The fourth and fifth group was immunised with two intramuscular doses (0, 14 days) of Ova (10 µg in 50 µL per animal) and three intranasal dose (0, 7 and 14 days) of Ova (20 µg in 25 µL per animal, 12.5 µL through each nostril) as antigen control without adjuvant For the evaluation of anti-OVA IgA levels were used feces obtained 21 days after the last dose. The determination was made by Ova IgA ELISA. The figure shows the average and standard deviation of the mathematical relationship of optical density values (OD) of 2 determinations in 3 independent experiments. The different p denote significant differences according to Tukey test (p <0.05).

## Claims

1. Vaccine formulations, **characterized by** containing proteoliposomes and/or its derivatives applied simultaneously by one or more immunization routes.

2. Vaccine formulations according to claim 1, **characterized by** the proteoliposomes and its derivatives are administered simultaneously by mucosal and parenteral rout.

3. Vaccine formulations according to claim 2, **characterized by** mucosal administration can be accomplished by either mucosal routes.

4. Vaccine formulations according to claims 1 and 2, **characterized** because they can be administered simultaneously in one of the following combinations: A + B, B + C, A + D or C + D:
A: proteoliposomes by mucosal route
B: proteoliposomes by parenteral route
C: derived from proteoliposomes by mucosal route D: derived from proteoliposomes by parenteral route

5. Vaccine formulations according to claims 2 to 4, **characterized by** mucosal administration can be intranasal, oral, sublingual, vaginal and / or rectum.

6. Vaccine formulations according to claims 2 and 4, wherein the parenteral administration can be intramuscular, subcutaneous, intradermal or transdermal.

7. Vaccine formulations according to claim 1, **characterized by** the proteoliposomes and its derivatives act as adjuvants for their own antigens (homologous) and/or unrelated antigens (heterologous).

8. Vaccine formulations according to claim 1, **characterized** because the derivatives of the proteoliposomes can be the cochleates.

9. Unitemporal Application of vaccine formulations where other mucosal adjuvants are used.

10. Unitemporal Application of vaccine formulations where the parenteral application can be amplified by the addition of other delivery systems.

11. Use of vaccine formulations by Unitemporal Application using two or more routes of administration

12. Use of vaccine formulations according to claim 11, comprising simultaneous application of proteoliposomes and its derivatives by two or more routes of administration using the Unitemporal Vaccination Strategy.

13. Use of vaccine formulations according to claims 11 and 12, which comprises the administration of proteoliposomes and its derivatives via Mucosal and Parenteral.

14. Use of vaccine formulations according to claim 13, wherein the mucosal administration can be accomplished by several ways.

15. Use of vaccine formulations according to claims 12, **characterized** because they can be administered simultaneously in one of the following combinations: A + B, B + C, A + D or C + D:
A: proteoliposomes by mucosal route
B: proteoliposomes by parenteral route
C: derived from proteoliposomes by mucosal route D: derived from proteoliposomes by parenteral route

16. Use of vaccine formulations according to claims 13 to 15, wherein the mucosal administration can be nasal, oral, sublingual, vaginal and / or rectum.

17. Use of vaccine formulations according to claims 13 and 15, wherein the parenteral administration can be intramuscular, intradermal, subcutaneous or transdermal.

18. Use of vaccine formulations according to claims 11 through 16, for the prevention and treatment of infections or tumor diseases.

19. Use of vaccine formulations according to claim 11, which apply different types of antigens by one or more mucosal route simultaneously with the corresponding combined vaccine by parenteral rout.

20. Treatment method **characterized by** the application of vaccine formulations by Unitemporal Vaccination Strategy using two or more routes of administration.

21. Treatment method according to claim 20, comprising simultaneous application of proteoliposomes and its derivatives by two or more routes of administration using the Unitemporal Vaccination Strategy.

22. Method of treatment according to claims 20 and 21, which comprises the simultaneous application of proteoliposomes and its derivatives by mucosal and parenteral rout.

23. Method of treatment according to claim 22, wherein the mucosal administration can be accomplished by several ways.

24. Method of treatment according to claim 22, **characterized** because they can be administered simultaneously in one of the following combinations: A + B, B + C, A + D or C + D:
A: proteoliposomes by mucosal route
B: proteoliposomes by parenteral route
C: derived from proteoliposomes by mucosal route D: derived from proteoliposomes by parenteral route

25. Method of treatment according to claims 22 to 24, wherein the mucosal administration can be nasal, oral, sublingual, vaginal and / or rectum.

26. Method of treatment according to claims 22 and 24, wherein the parenteral administration can be intramuscular, intradermal, subcutaneous or transdermal.

27. Method of treatment according to claims 20 to 26, for the prevention and treatment of infections and tumor diseases

28. Treatment method according to claim 20, where they apply different types of antigens by one or more mucosal route simultaneously with the corresponding combined vaccine by parenteral rout.
